# EUROPÄISCHE PATENTSCHRIFT

(11) **EP 2 956 104 B1**
(45) Veröffentlichungstag und Bekanntmachung des Hinweises auf die Patenterteilung: **11.01.2017**
(21) Anmeldenummer: 14704147.9
(22) Anmeldetag: 12.02.2014
(51) Int. Cl.: A61F 13/02, A61F 17/00, A61F 13/00

(54) **VERBANDSET ZUR BEHANDLUNG VON WUNDKAVITÄTEN**
DRESSING KIT FOR TREATING WOUND SINUSES
KIT DE PANSEMENT POUR LE TRAITEMENT DE CAVITÉS DE PLAIES

(30) Priorität: 13.02.2013 DE 102013002497
(43) Veröffentlichungstag der Anmeldung: 23.12.2015
(73) Patentinhaber: Paul Hartmann AG, 89522 Heidenheim (DE)
(72) Erfinder: CROIZAT, Pierre, 89542 Herbrechtingen (DE); ECKSTEIN, Axel, 89522 Heidenheim (DE); WOLF, Cornelia, 89542 Herbrechtingen (DE); EBIGBO, Njikoha, 89231 Neu-Ulm (DE)
(86) Internationale Anmeldenummer: PCT/EP2014/052734
(87) Internationale Veröffentlichungsnummer: WO 2014/124972

(56) Entgegenhaltungen:
- WO-A1-96/14038
- DE-A1-102008 061 536
- US-A1- 2004 209 041
- US-A1- 2009 093 779
- US-A1- 2011 172 617
- US-A1- 2012 157 945

## Beschreibung

Die vorliegende Erfindung betrifft ein Verbandset zur Verwendung bei der Behandlung von Wundkavitäten, insbesondere mittels Unterdruck, umfassend ein erstes Verbandmaterial als Wundkontaktschicht und ein separat bereit gestelltes zweites Verbandmaterial als Wundfüller.

Vorrichtungen zur Unterdrucktherapie von Wunden und Verbände als Bestandteil derartiger Vorrichtungen sind im Stand der Technik bekannt. So beschreibt beispielsweise die WO1993/009727 eine Vorrichtung zur Förderung der Wundheilung durch die Applikation von Unterdruck auf dem die Wunde aufweisenden und die Wunde umgebenden Hautbereich.

Bei der Unterdrucktherapie von Wunden kommuniziert eine Unterdruck erzeugende Einrichtung über eine Saugleitung mit der Wunde oder dem Wundraum, wobei ein luft- und unterdruckdichter Wundverband zum luft- und unterdruckdichten Verschließen der Wunde und des Wundraums vorgesehen ist, so dass ein Unterdruck im Wundraum herstellbar und Flüssigkeiten aus dem Wundraum in einen typischerweise zwischen der den Unterdruck erzeugenden Einrichtung und der Wunde angeordneten Behälter absaugbar sind. Der Ausdruck "Unterdruck" bezeichnet dabei einen innerhalb des Wundverbandes gegenüber dem Umgebungsluftdruck (atmosphärischer Luftdruck) erniedrigten Luftdruck. Unter "innerhalb des Wundverbandes" wird der durch das luftdichte Abdeckmaterial und dem Körpergewebe im Wundbereich gebildete Zwischenraum (Wundraum) verstanden. "Unterdruck" wird häufig auch als "negativer Druck" bezeichnet. Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d.h. die Druckdifferenz zwischen dem Luftdruck innerhalb des Wundverbandes und dem Umgebungsluftdruck, als positiver Zahlenwert in mm Hg angegeben.

Die Unterdrucktherapie kann zur Behandlung einer Vielzahl unterschiedlicher Wundtypen eingesetzt werden, beispielsweise Druckwunden (Dekubitus), Geschwüren (Ulcus), Brandwunden oder Wunden infolge traumatischer Einwirkung. Insbesondere findet die Unterdrucktherapie Anwendung bei der Behandlung schlecht heilender und/oder infizierter Wunden.

Wunden mit komplexer Wundgeometrie können Wundkavitäten umfassen. Im Zusammenhang mit der vorliegenden Erfindung wird unter dem Ausdruck "Wundkavität" ein Wundbereich mit subdermaler Ausdehnung verstanden, beispielsweise eine Wundtasche oder Wundröhre, wobei die Wundröhre blind endend oder durchgehend (Wundtunnel) ausgebildet sein kann. In der englischen Terminologie werden Wundkavitäten auch als "wound sinus" oder "sinus tract" bezeichnet.

Wundbereiche mit subdermaler Ausdehnung können auf eine Vielzahl unterschiedlichster Ursachen zurückgehen. Die Entstehung einer Wundkavität kann beispielsweise im Zusammenhang mit einem entzündlichen (inflammatorischen) Prozess stehen. Inflammatorische Prozesse sind üblicherweise an der Pathogenese chronischer oder verzögert heilender Wunden beteiligt. Der Entzündungsprozess kann zum nekrotischen Abbau von Gewebe und damit zur Ausbildung von Hohlräumen im Wundbereich führen. Ebenso können lokale Infektionen, welche beispielsweise infolge einer Implantation von medizinischen Geräten und Prothesen auftreten, zu einer Entzündung und nachfolgend zur Ausbildung tiefer Wundtaschen beitragen. Wundkavitäten können auch infolge traumatischer Verletzungen, beispielsweise durch Décollement, Quetschung, Avulsion oder ballistisches Trauma entstehen. Durch die während des Unfalls eingetragenen Mikroorganismen besteht zudem ein hohes Infektionsrisiko, welches die Gefahr eines zusätzlichen, inflammatorisch bedingten Gewebeverlusts mit sich bringt.

Zur Exploration einer Wundkavität ist häufig eine Sondierung der Hohlräume erforderlich. Die Versorgung einer Wunde, welche eine oder mehrere Wundkavitäten umfasst, kann die Tamponade der Wundhöhle mit Schäumen, Mullprodukten oder Alginaten umfassen. Bei hohem Exsudataufkommen wird die Flüssigkeit über Drainagen aus der Wunde abgeführt. Die Auskleidung des Wundbetts mit Füllmaterialien verhindert die Austrocknung des Wundbetts und unterstützt die Ausbildung von funktionellem Granulationsgewebe. Weiterhin soll die Bildung unerwünschter Granulationsbrücken verhindert werden, da diese zur Ausbildung abgekapselter Hohlräume führen können. Schwierigkeiten ergeben sich häufig beim Entfernen der als Tamponade eingesetzten Materialien.

Die Unterdrucktherapie stellt eine neue Behandlungsoption zur Behandlung von Wunden mit komplexer Wundgeometrie dar. Hierzu kann ein Schaumstoffmaterial in die Kavität eingebracht und der Wundraum mit einer Unterdruckquelle verbunden werden, um die Bildung von Granulationsgewebe zu fördern und Wundexsudat abzuführen. Der Schaumstoff wird im Rahmen aufeinanderfolgender Behandlungszyklen sukzessive verkürzt. Allerdings besteht bei dieser Behandlungsmethode die Gefahr, insbesondere bei der Behandlung tiefer, enger Wundhöhlen, dass beim Verbandwechsel Schaumstoffreste zurückbleiben. Zudem hat sich gezeigt, dass das Schaumstoffmaterial unter dem Einfluss des Unterdrucks kollabieren kann, so dass der Abfluss von Wundexsudat aus den Wundtaschen behindert wird (Sekretstau). Zur Vermeidung eines Sekretstaus wurde vorgeschlagen, in ausgedehnte Wundhöhlen Drainageleitungen (Redondrainage) einzubringen, so dass das Wundexsudat ungehindert abgeleitet werden kann. Gleichzeitig wird der zentrale offene Bereich der Wunde, in welchen die Kavitäten einmünden, mit einem Schaumstoff ausgekleidet. Die Drainageleitungen werden vom Unterdruckanschlussstück durch den Schaumstoff im zentralen offenen Bereich der Wunde hindurch zu den Wundhöhlen gelegt, so dass die Kavitäten offen gehalten und darin enthaltene Flüssigkeit abgesaugt werden kann (Grimm, Loos und Horch in Zentralbl Chir 2006; 131: S19-23). Wenn der Wundgrund, also die Wandungen der Kavität, während der Applikation von Unterdruck allerdings gegen die Drainageleitung gedrückt wird, kann dies zu einem Verstopfen der Öffnungen der Drainageleitung führen.

Die WO2004/018020 beschreibt ein flexibles Wundinsert für die Unterdrucktherapie, welches in Wundröhren oder unterminierte Bereiche eingebracht werden kann. Das flächenartig ausgebildete Wundinsert, welches bei Bedarf zu einem Leitungselement aufgerollt werden kann, weist eine Vielzahl von Öffnungen und optional vorhandenen Kanälen auf. Durch das Einbringen des Wundinserts in eine Wundkavität soll ein vorzeitiger Wundverschluss und/oder die Abkapselung von Hohlräumen infolge der Bildung von Granulationsbrücken vermieden werden.

Die DE102008061536 offenbart einen Saugkörper für die endoluminale Unterdrucktherapie mit einer vorzugsweise entfernbaren Umhüllung, welche den Saugkörper in einer komprimierten Form zusammenhält. Optional kann die Umhüllung Perforationen, insbesondere Perforationen entlang einer Perforationslinie, aufweisen. Die Perforationslinie dient der Öffnung der Hülle, damit diese aus der betroffenen Körperhöhle entfernt werden kann.

Aus der medizinischen Praxis hat sich die Anforderung ergeben, eine verbesserte Wundauflage für Wundkavitäten zur Verfügung zu stellen. Eine derartige Wundauflage sollte die Kavität möglichst vollständig auskleiden, ohne jedoch mit dem Wundgrund zu verkleben oder zu verwachsen. Als Anforderung von herausragender Bedeutung hat sich ergeben, dass beim Verbandwechsel keinerlei Materialrückstände in den schwer zugänglichen Hohlräumen der Wunde verbleiben dürfen. Zurückgebliebene Materialstücke können zu schweren Komplikationen nach der Behandlung führen, beispielsweise zur Ausbildung von Abszessen. Weiterhin soll die Wundauflage den Exsudatabfluss auch während der Unterdrucktherapie ungehindert und vollständig gewährleisten. Insgesamt sollte die Wundauflage flexibel einsetzbar und durch den Benutzer an die jeweilige Wundsituation anpassbar sein. Sie sollte weiterhin einfach anzulegen und in möglichst wenig Arbeitsschritten entfernbar sein.

Die vorliegende Erfindung schlägt zur Versorgung von Wunden, welche Wundkavitäten aufweisen, ein Verbandset sowie einen unter Verwendung des Verbandsets herstellbaren, mindestens zwei Komponenten umfassenden Verbund vor.

Erfindungsgemäß umfasst das Verbandset, welches insbesondere, jedoch nicht ausschließlich, zur Verwendung bei der Behandlung von Wundkavitäten mittels Unterdruck geeignet ist, ein erstes Verbandmaterial als Wundkontaktschicht. Die Wundkontaktschicht umfasst eine flexible, perforierte Folie mit einer ersten und einer zweiten Seite. Die in der Folie vorhandenen Perforationen sind derart beschaffen beziehungsweise werden derart in die Folie eingebracht, dass die Perforationsränder von der zweiten Seite der Folie abstehen. Die von der zweiten Seite der Folie abstehenden Perforationsränder bilden somit dreidimensional ausgestaltete Strukturen, welche auf der zweiten Seite der Folie vorhanden sind. Die erste Seite der Folie ist zum Inkontaktbringen mit einem Wundgrund, insbesondere zum Inkontaktbringen mit der inneren Oberfläche einer Wundröhre, vorgesehen. Das Verbandset umfasst weiterhin ein separat bereit gestelltes, zweites Verbandmaterial zum Einbringen in eine Wundröhre, welches somit als Wundfüller vorgesehen ist. Das zweite Verbandmaterial umfasst einen porösen Polymerschaum, wobei es sich bei dem Polymerschaum um einen offenzelligen Polymerschaum handelt, welcher an oder nahe seiner Oberfläche Stege umfasst und/oder welcher auf seiner Oberfläche zur Oberfläche hin offene Hohlräume umfasst. Die Stege und/oder Hohlräume bilden hierbei dreidimensional ausgestaltete Strukturen. Erfindungsgemäß ist erforderlich, dass das erste Verbandmaterial über eine Fläche verfügt, welche ausreichend ist, mindestens 75 %, vorzugsweise mindestens 85 %, insbesondere mindestens 95 % der Oberfläche des zweiten Verbandmaterials zu umhüllen. Erfindungsgemäß ist weiterhin erforderlich, dass die auf der zweiten Seite des ersten Verbandmaterials vorhandenen Strukturen mit den auf der Oberfläche des zweiten Verbandmaterials vorhandenen Strukturen eine haftende Verbindung eingehen können. Des Weiteren ist erfindungsgemäß vorgesehen, dass zum Verschieben des ersten Verbandmaterials im nassen Zustand gegen das zweite Verbandmaterial im nassen Zustand eine statische Gleitreibungskraft Fₛ, gemessen nach DIN EN ISO 8235, von mindestens 3 N und/oder zum Verschieben des ersten Verbandmaterials im trockenen Zustand gegen das zweite Verbandmaterial im trockenen Zustand eine statische Gleitreibungskraft Fₛ von mindestens 6 N, gemessen nach DIN EN ISO 8235, erforderlich ist.

Die zweite Seite der Folie ist zum teilweisen oder vollständigen Umhüllen der Oberfläche des Polymerschaumstoffs unmittelbar vor dem Anlegen der Wundauflage beziehungsweise unmittelbar vor der Wundbehandlung vorgesehen, so dass ein Verbund aus erstem Verbandmaterial und zweitem Verbandmaterial entsteht. Der Verbund wird also durch den Anwender unmittelbar vor der Wundbehandlung hergestellt, wobei der Anwender die Größe und Gestalt der Komponenten durch Zuschneiden an die Wundverhältnisse anpassen kann. Nach dem Einbringen des Verbundes aus erstem und zweitem Verbandmaterial in eine Wundkavität kann aufgrund der haftenden Verbindung zwischen dem ersten Verbandmaterial und dem zweiten Verbandmaterial ein Verschieben des ersten Verbandmaterials gegenüber dem zweiten Verbandmaterial während der Therapie weitestgehend vermieden und/oder das gleichzeitige Entfernen von dem ersten Verbandmaterial und dem zweitem Verbandmaterial erleichtert werden.

Der mit der Erfindung vorgeschlagene Verbund, welcher mindestens zwei Komponenten umfasst, ist gleichfalls zur Verwendung bei der Behandlung von Wundkavitäten, insbesondere zur Verwendung bei der Behandlung von Wundkavitäten mittels Unterdruck, vorgesehen und geeignet. Mit dem erfindungsgemäßen Verbund wird dem Anwender, also üblicherweise dem behandelnden Arzt, eine speziell zur Behandlung von Wundkavitäten angepasste Wundauflage zur Verfügung gestellt. Der Verbund umfasst ein erstes Verbandmaterial als Wundkontaktschicht. Das erste Verbandmaterial umfasst hierbei eine flexible, perforierte Folie mit einer ersten und einer zweiten Seite, wobei die in der Folie vorhandenen Perforationen derart beschaffen sind, dass die Perforationsränder von der zweiten Seite der Folie abstehen, so dass auf der zweiten Seite der Folie dreidimensional ausgestaltete Strukturen vorhanden sind. Die erste Seite ist zum Inkontaktbringen mit einem Wundgrund, insbesondere mit der inneren Oberfläche einer Wundröhre, vorgesehen. Der Verbund umfasst des Weiteren ein zweites Verbandmaterial zum Einbringen in eine Wundkavität. Das zweite Verbandmaterial umfasst einen porösen Polymerschaum, wobei es sich bei dem Polymerschaum um einen offenzelligen Polymerschaum handelt, welcher an oder nahe seiner Oberfläche Stege umfasst und/oder welcher auf seiner Oberfläche zur Oberfläche hin offene Hohlräume umfasst, und wobei die Stege und/oder Hohlräume dreidimensional ausgestaltete Strukturen bilden. Wesentlich ist, dass die auf der zweiten Seite des ersten Verbandmaterials vorhandenen Strukturen mit den auf der Oberfläche des zweiten Verbandmaterials vorhandenen Strukturen eine haftende Verbindung eingehen können. Weiterhin muss die Folie auf mindestens 75 %, vorzugsweise auf mindestens 85 %, insbesondere auf mindestens 95 % der Oberfläche des Polymerschaums vorliegen. Die Folie bildet somit eine Umhüllung des Polymerschaums, wobei die Folie mit ihrer zweiten Seite in Kontakt mit dem Polymerschaum steht. Hierdurch wird erreicht, dass nach dem Einbringen des Verbundes aus erstem und zweitem Verbandmaterial in eine Wundkavität aufgrund der haftenden Verbindung zwischen dem ersten Verbandmaterial und dem zweiten Verbandmaterial ein Verschieben des ersten Verbandmaterials gegenüber dem zweiten Verbandmaterial während der Therapie weitestgehend vermieden werden kann und/oder dass das gleichzeitige Entfernen von dem ersten Verbandmaterial und dem zweitem Verbandmaterial erleichtert wird.

Der Verbund aus erstem und zweitem Verbandmaterial (im Folgenden auch als "Wundauflage" bezeichnet) kann in Kombination mit an sich aus dem Stand der Technik bekannten Verbandkomponenten verwendet werden. Insbesondere eignet sich die Wundauflage zur Anwendung in Kombination mit normalerweise zur Unterdrucktherapie von Wunden verwendbaren Komponenten, beispielsweise mit einem weiteren porösen Wundfüller, mit einer luftdichten selbstklebenden Abdeckfolie, mit einem Unterdruckanschlussmittel, mit einem Sekretbehälter und mit einer Unterdruckquelle.

Der erfindungsgemäße Verbund ermöglicht eine verbesserte Versorgung von Wunden mit Wundkavitäten, insbesondere bei Anwendung der Unterdrucktherapie. Die neuartige Wundauflage ermöglicht demnach insbesondere eine verbesserte Behandlung von Wundkavitäten aufweisenden Wunden mittels Unterdruck, wobei die Wundheilung unterstützt, die Behandlung erleichtert und der Patient vor unerwünschten Komplikationen bewahrt wird. Die mit der Wundauflage erreichbaren Verbesserungen und Vorteile gegenüber dem Stand der Technik betreffen also den positiven Heilungsverlauf der Unterdrucktherapie, die Handhabbarkeit der Wundauflage für den behandelnden Arzt ebenso wie die Vermeidung von Nebenwirkungen aus der Unterdrucktherapie.

Eine wesentliche Verbesserung bei der Versorgung von Wunden, welche Bereiche mit Kavitäten umfassen, ergibt sich dadurch, dass die erfindungsgemäße Wundauflage durch den Anwender, beispielsweise durch den behandelnden Arzt, unmittelbar vor der Wundbehandlung an die spezifische Wundsituation angepasst werden kann. Aufgrund der Anpassung des Verbundes an die Abmessungen der zu behandelnden Wundkavität kann die Kavität vollständig durch die Wundauflage ausgefüllt, ohne dass Hohlräume, in denen sich Wundexsudat sammeln kann, zurückbleiben. Weiterhin ist die Wundkontaktschicht während der Behandlung durchgehend in Kontakt mit dem Wundgrund, welcher durch die Wandungen der Kavität gebildet wird. Eine weitestgehende Abdeckung des Wundgrundes während des Vorhandenseins von Unterdruck kann die Ausbildung von Granulationsgewebe fördern.

In der Praxis erweist sich der durch Haftkräfte zusammengehaltene und in die Wundkavität eingebrachte Verbund aus einer perforierten Folie, welche dreidimensionale Strukturen aufweist, mit einem porösen Polymerschaum in mehrerlei Hinsicht als besonders vorteilhaft. Einerseits können die in der Folie vorhandenen dreidimensional ausgebildeten Perforationen einen Kapillareffekt ausbilden, welcher eine effektive Ableitung des Wundexsudates vom Wundgrund durch die Folie hindurch zum Schaum bewirken kann. Der offenzellige Polymerschaum wiederum gewährleistet über seine inneren Hohlräume einen ungehinderten Weitertransport der Flüssigkeit hin zur Wundöffnung. Hinsichtlich der Abmessungen der Perforationen hat sich ein offener Durchmesser von vorzugsweise mindestens 100 µm und höchstens 1000 µm, besonders bevorzugt mindestens 300 µm und höchstens 500 µm als vorteilhaft erwiesen, da bei einer derartigen Dimensionierung einerseits ein hinreichender Kapillareffekt vorhanden ist und anderseits eine ausreichende Permeabilität durch die an sich Fluid-undurchlässige Folie gewährleistet ist. Die Höhe der dreidimensional ausgestalteten Strukturen (maximale Erstreckung der dreidimensionalen Struktur von der Planebene der Folie bis zu den Perforationsrändern) sollte dabei im Zusammenhang mit dem Kapillareffekt vorzugsweise 100 µm bis 2000 µm betragen, besonders bevorzugt 300 µm bis 700 µm.

Anderseits ergibt sich durch die Umhüllung des Schaums mit einer Folie eine Stabilisierung des Polymerschaumstoffs in engen Wundröhren, so dass ein Kollabieren des Schaums in Gegenwart von Unterdruck weitgehend vermieden werden kann. Dieser Effekt trägt dazu bei, dass das Wundexsudat während der gesamten Dauer der Therapie auch aus engen Kavitäten abgeleitet wird.

Des Weiteren stellt die auf ihrer ersten Seite eine weitgehend glatte Oberfläche aufweisende Folie eine atraumatische Wundkontaktschicht zur Verfügung, welche keine Neigung zeigt, mit dem frisch gebildeten Granulationsgewebe zu verwachsen.

Weiterhin kann der durch Haftkräfte zusammengehaltene Verbund aus einer perforierten Folie mit dem porösen Polymerschaum nach Beendigung der Therapie in einem Schritt entfernt werden.

Schließlich gewährleistet die den Schaum umhüllende Folie, dass keine Schaumstoffstücke in den Kavitäten zurückbleiben, so dass eine wesentliche, bei der Behandlung von Kavitäten häufig auftretende Komplikation weitestgehend vermieden werden kann. Die Sicherheit einer Unterdruckbehandlung von Wundkavitäten lässt sich hierdurch erheblich verbessern.

Der Verbund aus einer perforierten Folie, welche dreidimensionale Strukturen aufweist, mit einem porösen offenzelligen Polymerschaum, welcher an oder nahe seiner Oberfläche Stege umfasst und/oder welcher auf seiner Oberfläche zur Oberfläche hin offene Hohlräume umfasst, und wobei die Stege und/oder Hohlräume dreidimensional ausgestaltete Strukturen bilden, wird erfindungsgemäß durch Haftkräfte zusammengehalten. Die hierfür erforderlichen Haftkräfte müssen hinreichend stark sein, den Verbund während des Einbringens in die Kavität, weiter während der Dauer der Wundbehandlung und schließlich während des Herausziehens der Wundauflage beim Verbandwechsel zusammen zu halten. Erfindungswesentlich ist, dass der Verbund sowohl im trockenen Zustand (beispielsweise beim Applizieren der Wundauflage) als auch im nassen Zustand (beispielsweise während der Therapie oder nach Beendigung der Therapie) durch hinreichend starke Haftkräfte zusammengehalten wird.

Bei der Auswahl geeigneter Materialien für das erste und das zweite Verbandmaterial wird die Gleitreibungskraft Fₛ, gemessen nach DIN EN ISO 8235, zur Abschätzung der zwischen erstem und zweitem Verbandmaterial vorhandenen Haftkraft herangezogen. Hierzu wird die zwischen erstem und zweitem Verbandmaterial auftretende Gleitreibungskraft Fₛ nach dem im Ausführungsbeispiel dargestellten Verfahren, welches auf der DIN EN ISO 8235 beruht, bestimmt. Vorzugsweise erfolgt die Bestimmung der Gleitreibungskraft Fₛ sowohl im trockenen als auch im nassen Zustand.

Erfindungsgemäß ist zum Verschieben des ersten Verbandmaterials im nassen Zustand gegen das zweite Verbandmaterial im nassen Zustand eine statische Gleitreibungskraft Fₛ, gemessen nach DIN EN ISO 8235, von mindestens 3 N erforderlich und/oder es ist zum Verschieben des ersten Verbandmaterials im trockenen Zustand gegen das zweite Verbandmaterial im trockenen Zustand eine statische Gleitreibungskraft Fₛ von mindestens 6 N, gemessen nach DIN EN ISO 8235, erforderlich. Bevorzugt ist zum Verschieben des ersten Verbandmaterials im nassen Zustand gegen das zweite Verbandmaterial im nassen Zustand eine statische Gleitreibungskraft Fₛ, gemessen nach DIN EN ISO 8235, von mindestens 5 N erforderlich und/oder es ist zum Verschieben des ersten Verbandmaterials im trockenen Zustand gegen das zweite Verbandmaterial im trockenen Zustand eine statische Gleitreibungskraft Fₛ von mindestens 8 N, gemessen nach DIN EN ISO 8235, erforderlich.

Da die zwischen den Oberflächen vorhandene Gleitreibungskraft herstellungsbedingt in Längsrichtung ("Maschinenrichtung", auch als "MD" bezeichnet) und Querrichtung der Materialien variieren kann, wird hier unter einer "mindestens erforderlichen statischen Gleitreibungskraft Fₛ" die bei unterschiedlichen Orientierungen der Lagen zueinander geringste auftretende statische Gleitreibungskraft Fₛ verstanden. Hierdurch kann gewährleitet werden, dass der Verbund aus Folie und Polymerschaum sowohl im trockenen Zustand als auch im nassen Zustand durch hinreichend starke Haftkräfte zusammengehalten wird.

Das erste Verbandmaterial umfasst eine flexible, perforierte Folie mit einer ersten und einer zweiten Seite, als Wundkontaktschicht.

Es ist wesentlich, dass die Wundkontaktschicht aus einem Material besteht, welches während der Dauer der Anwendung nicht mit dem Wundgrund verklebt oder verwächst. Das Material soll deshalb atraumatische Eigenschaften aufweisen. Bevorzugt umfasst das erste Verbandmaterial eine thermoplastische Folie. Geeignete Materialien für eine thermoplastische Folie umfassen insbesondere Ethylvinylacetat (EVA), Polyurethan (PU), Polyethylen (PE), Polyethylenterephthalat (PET), Polytetrafluorethylen (PTFE), Polyvinylchlorid (PVC), thermoplastische Elastomere (TPE), Polyorganosiloxan (Silikon) oder einer Mischung daraus. Unter der Bezeichung TPE sind in diesem Zusammenhang thermoplastische Elastomere auf Olefinbasis (TPO), vernetzte thermoplastische Elastomere auf Olefinbasis (TPV), thermoplastische Elastomere auf Urethanbasis (TPU), thermoplastische Polyesterelastomere bzw. thermoplastische Copolyester (TPC), Styrol-Blockcopolymere (TPS) und thermoplastische Copolyamide (TPA) umfasst. Vorzugsweise handelt es sich bei der thermoplastischen Folie um eine PE-Folie.

Das Flächengewicht der Folie sollte mindestens 30 g / m² und höchstens 150 g / m², vorzugsweise mindestens 45 g / m² und höchstens 95 g / m² und insbesondere mindestens 55 g / m² und höchstens 65 g / m² betragen.

Die als Wundkontaktschicht vorgesehene flexible Folie wird vor der Applikation der Wundauflage auf das einen Polymerschaum umfassende zweite Verbandmaterial aufgebracht oder um das zweite Verbandmaterial gewickelt. Die flexible Folie bildet somit eine das zweite Verbandmaterial teilweise oder vollständig bedeckende Hülle.

Normalerweise liegt die Wundkontaktschicht überwiegend als einlagige Materialschicht auf dem Polymerschaum vor. Es ist jedoch auch möglich, mehr als eine Folienlage übereinander auf den Schaum aufzubringen. Insbesondere ergibt sich beim Umwickeln des Schaums mit der Folie im Überlappungsbereich der Folienbahnen häufig ein mindestens zweilagiger Bereich. Die Materialstärke der Folie beträgt (ohne Berücksichtigung der an der zweiten Oberfläche vorhandenen dreidimensionalen Strukturen) 10 bis 1000 µm, vorzugsweise 10 bis 500 µm.

Das zweite Verbandmaterial umfasst einen offenzelligen porösen Polymerschaumstoff, insbesondere einen Schaum aus Polyurethan, einen Schaum aus Silikon oder einen Schaum aus Polyvinylalkohol. Besonders bevorzugt umfasst das zweite Verbandmaterial einen Schaum aus Polyester-Polyurethan.

Hinsichtlich seiner äußeren Form liegt das zweite Verbandmaterial vorzugsweise in Form eines Quaders oder in Form eines Zylinders vor. Ein Zylinder mit einem im Vergleich zu seiner Höhe (h) geringem Durchmesser (d), also ein schlauchförmiger ausgebildeter Zylinder, eignet sich besonders zum Einbringen in eine enge Wundröhre. Besonders bevorzugt liegt das zweite Verbandmaterial in Form eines schlauchförmigen Zylinders vor, bei dem das Verhältnis von Höhe (h) zu Durchmesser (d) mindestens 3, insbesondere mindestens 5, ganz besonders bevorzugt mindestens 10 beträgt.

Für den Anwender kann es sich als sehr praktisch erweisen, wenn das zweite Verbandmaterial dem Anwender in Form einer Auswahl schlauchförmiger Schaumstoffabschnitte (deren Länge beispielsweise jeweils 10 cm betragen könnte) mit unterschiedlichen Durchmessern zur Verfügung gestellt wird. Die Durchmesser könnten beispielsweise 1 cm, 2 cm, 3 cm, 4 cm und 5 cm betragen. Der Anwender kann dann nach der Exploration der Wunde einen Schaumstoff mit einem für die zu behandelnde Kavität geeignetem Durchmesser auswählen.

Gemäß einer weiteren bevorzugten Ausführungsform liegt das zweite Verbandmaterial in Form eines Quaders mit den Seiten a; b; c vor, insbesondere in Form eines in die Länge gestreckten Quaders mit einem Verhältnis der Seitenlängen der durch die Seiten (a; b) gebildeten Querschnittsfläche von 0,5 < (a : b) < 2, wobei die längere Seite c länger als die Diagonale der Seitenfläche (a; b) sein soll. Besonders bevorzugt weist der Quader ein annähernd quadratisches Verhältnis der Seitenlängen von 0,9 < (a : b) < 1,1 auf, wobei die längere Seite c mindestens das doppelte, insbesondere mindestens das fünffache der Diagonale der Seitenfläche (a; b) aufweisen soll. Gemäß dieser besonders bevorzugten Ausführungsform ergibt sich ein in die Länge gestreckter Schaumstoffblock mit einem annähernd (im Falle von 0,9 < (a : b) < 1,1) oder vollständig (im Falle von a = b) quadratischen Querschnitt.

Analog zu den vorstehend beschriebenen, schlauchförmig ausgestalteten Schaumstoffabschnitten, kann es sich für den Anwender auch hier als sehr praktisch erweisen, wenn das zweite Verbandmaterial in Form einer Auswahl von Schaumstoffabschnitten (deren Länge beispielsweise jeweils 10 cm betragen könnte) mit unterschiedlichen Durchmessern zur Verfügung gestellt wird. Die Durchmesser (Länge einer Seite a im Falle eines quadratischen Querschnittes) könnten beispielsweise 1 cm, 2 cm, 3 cm, 4 cm und 5 cm betragen.

Im Rahmen der Erfindung kann das zweite Verbandmaterial auch in weiteren, hier nicht näher beschriebenen Ausgestaltungen vorliegen. Für die medizinische Anwendung ist es vorteilhaft, wenn das zweite Verbandmaterial bereits herstellerseitig eine äußere Form aufweist, welche die Anpassung an eine zu behandelnde Kavität erleichtert.

Die Anpassung des zweiten Verbandmaterials an eine zu behandelnde Kavität erfolgt normalerweise durch Zuschneiden, beispielsweise mit einer sterilisierten Schere oder mit einem sterilisierten Skalpell.

Das zweite Verbandmaterial kann einlagig ausgebildet sein oder mehrere Schichten umfassen. Im Falle einer mehrere Schichten umfassenden zweiten Verbandlage ist es möglich, dass die Schichten lösbar oder unlösbar miteinander verbunden sind. Ein Zusammenhalt der Schichten kann beispielsweise durch ein Umwickeln der Lagen mit einem weiteren Material und/oder mit der ersten Verbandlage hergestellt werden. Im Falle einer mehrere Schichten umfassenden, zylinderförmig ausgebildeten zweiten Verbandlage kann eine konzentrische Anordnung der Schichten günstig sein. Optional kann das zweite Verbandmaterial eine oder mehrere zusätzliche Schichten eines textilen Materials wie Woven oder Non-woven umfassen, beispielsweise einen Vliesstoff aus synthetischen Polymeren wie Polyamid, Polyester oder Polypropylen. So wäre beispielsweise denkbar, dass ein zylinderförmig ausgebildetes zweites Verbandmaterial einen Mantel aus einem offenzelligen Polymerschaumstoff und einen Kern aus einem Non-woven umfasst.

Erfindungsgemäß umfasst das zweite Verbandmaterial einen offenzelligen porösen Polymerschaumstoff. Eine Zelle ist der bei der Herstellung von Schaumstoffen gebildete einzelne Hohlraum, der von Zellwänden und/oder Zellstegen teilweise oder vollständig umschlossen ist. Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Schaumstoffs wird üblicherweise nach ASTM D 2856-87, Verfahren B) bestimmt. Unter Zellwand wird üblicherweise die die Zelle umschließende Wand verstanden. Die Zellwand kann auch als Zellmembran bezeichnet werden. Als Zellsteg oder Steg wird üblicherweise der Bereich der Zellwand verstanden, der mehr als zwei Zellen voneinander trennt. Bei dem im Zusammenhang mit der Erfindung verwendbaren offenzelligen Schaumstoff kann es sich um einen retikulierten oder um einen nichtretikulierten Schaumstoff handeln. Unter einem retikulierten Schaumstoff wird ein Schaumstoff verstanden, der im Wesentlichen nur Stege aufweist. Bei einem retikulierten Schaumstoff sind die Zellwände somit im Wesentlichen entfernt.

Erfindungswesentlich ist vorgesehen, dass auf der zweiten Seite des ersten Verbandmaterials vorhandene Strukturen mit auf der Oberfläche des zweiten Verbandmaterials vorhandenen Strukturen eine haftende Verbindung eingehen können. Entsprechend müssen auf der Oberfläche der zweiten Seite des ersten Verbandmaterials und auf der Oberfläche des zweiten Verbandmaterials dreidimensional ausgestaltete Strukturen (auch als 3-D-Strukturen bezeichnet) vorhanden sein, welche eine Haftung zwischen den beiden Oberflächen bewirken. Bei der Haftung handelt es sich insbesondere um eine auf Formschluss oder Kraftschluss beruhende Haftung.

Bei den auf der zweiten Oberfläche des ersten Verbandmaterials vorhandenen dreidimensional ausgestalteten Strukturen handelt es sich um in die Folie eingebrachte Perforationen, wobei die Perforationsränder von der zweiten Seite der Folie abstehen. Die 3-D-Strukturen ragen hierbei ausschließlich aus der zweiten Oberfläche heraus, so dass die zweite Oberfläche eine raue Beschaffenheit aufweist, während die erste Oberfläche, welche zum Inkontaktbringen mit einem Wundgrund vorgesehen ist, eine weitestgehend glatte Beschaffenheit aufweist.

Die in die Folie eingebrachten Perforationen können beispielsweise kraterartig aus der Planebene der Folie herausragen, so dass eine Mikrostruktur entsteht, welche mit einem geeigneten Widerpart an der Oberfläche des zweiten Verbandmaterials eine haftende Verbindung eingehen kann.

Bei den eine Haftung ermöglichenden dreidimensional ausgestalteten Strukturen, welche im offenzelligen Polymerschaum vorhanden sind, handelt es sich um die Stege der Schaumzellen und/oder um zur Oberfläche des Schaumes hin offene Hohlräume.

Um den erwünschten Hafteffekt zu erzielen, muss bei der Auswahl des Verbandmaterials zur Herstellung der zweiten Verbandlage darauf geachtet werden, dass ein ausreichender Anteil an Schaum-Stegen an oder nahe der Schaumoberfläche vorhanden und somit dem Zugriff haftender Mikrostrukturen des ersten Verbandmaterials ausgesetzt ist und/oder dass eine ausreichende Anzahl zur Oberfläche des Schaumes hin offener Hohlräume vorhanden ist. Die Stege sind im Wesentlichen innerhalb des Schaumstoffkörpers vorhanden, so dass sie üblicherweise nicht über die Ebene der dreidimensional strukturieren Schaumoberfläche herausstehen. Eine hakende oder anderweitige eine Haftung bewirkende Wechselwirkung der auf der Oberfläche des ersten Verbandmaterials vorhandenen dreidimensionalen Strukturen mit den Mikrostrukturen auf der Oberfläche des zweiten Verbandmaterials, namentlich die Perforationsränder und die Stege des Schaums, kann nur stattfinden, wenn die beteiligten Strukturelemente hinsichtlich ihrer Abmessungen aufeinander abgestimmt sind. Eine haftende Interaktion mit den auf dem ersten Verbandmaterial vorhandenen dreidimensional ausgestalteten Mikrostrukturen kann alternativ oder zusätzlich, also in einer die Haftung verstärkenden Weise, durch eine Vielzahl von zur ersten Oberfläche des Schaumes hin offenen Hohlräumen erreicht werden, in welche die auf der zweiten Seite der Folie vorhandenen Mikrostrukturen eindringen können, so dass eine form- und/oder kraftschlüssige Verbindung entsteht. Auf mikroskopischer Ebene betrachtet, weist die Oberfläche eines offenzelligen Polymerschaums, welcher eine Vielzahl zur Oberfläche des Schaumes hin offener Hohlräume umfasst, eine dreidimensional ausgestalte Oberflächenstruktur auf. Bei den zur Oberfläche hin offenen Hohlräumen handelt es sich um im Inneren des Schaumstoffes vorhandene Zellen, welche durch einen bei der Herstellung der Verbandlage erforderlichen Zuschnitt des Schaumstoffes teilweise freigelegt werden. Um eine haftende Interaktion zwischen den an der Folie vorhandenen Mikrostrukturen und den am Schaumstoff vorhandenen Hohlräumen zu erzielen, ist es gleichfalls erforderlich, dass die an beiden Verbandlagen vorhandenen Mikrostrukturen und die zur Oberfläche des Schaums hin offenen Hohlräume hinsichtlich ihrer Abmessungen aufeinander abgestimmt sind.

Wie bereits dargestellt, erweist es sich hier als besonders vorteilhaft, wenn auf der zweiten Oberfläche der Folie insbesondere eine kraterförmig geformte Struktur vorhanden ist. Hierbei kommt es darauf an, dass die kraterförmige Struktur in einem die Haftung ermöglichendem Maße über die plane Oberfläche der Folie hinausragt. Allerdings sollte die Struktur nicht allzu weit aus der Oberfläche herausragen, da ansonsten die Festigkeit der hakenden Mikrostrukturen nachlassen kann. Die kraterförmige Struktur weist deshalb vorzugsweise eine Höhe von mindestens 100 µm und höchstens 2000 µm, insbesondere von mindestens 200 µm und höchstens 1000 µm und ganz besonders bevorzugt von mindestens 300 µm und höchstens 700 µm auf. Unter Höhe wird hier die senkrecht zur Ebene der Folie gemessene maximale Erstreckung der dreidimensionalen Struktur verstanden. Die Messung kann anhand geeigneter mikroskopischer Aufnahmen von Folienquerschnitten ermittelt werden. Weiterhin wird die Haftungswirkung der durch die Perforation gebildeten Mikrostrukturen vom Winkel beeinflusst, der durch die Ebene der Kraterwände gegen die Ebene der planen Folie gebildet wird. Hier hat es sich gezeigt, dass eine wirksame Haftung insbesondere dann beobachtbar ist, wenn der vorgenannte Winkel mindestens 10° und höchstens 90°, vorzugsweise mindestens 45° und höchstens 80° beträgt.

Gemäß einer im Rahmen der Erfindung besonders vorteilhaften Ausführungsform erweist es sich hinsichtlich der Haftung als besonders vorteilhaft, wenn das erste und das zweite Verbandmaterial folgende Merkmale in Kombination aufweist:
a) Das erste Verbandmaterial (Wundkontaktschicht) umfasst eine transparente, dreidimensional perforierte Folie aus Polyethylen. Die Perforationsränder ragen aus der zweiten Seite der Folie kraterförmig hervor. Die Höhe der auf der zweiten Seite vorhandenen dreidimensionalen trichterförmigen Struktur beträgt 300 µm bis 700 µm. Die Dicke des Folienmaterials (Materialstärke ohne Berücksichtigung der Perforationen) beträgt 10 µm bis 100 µm. Die offene Fläche der in der Folie vorhandenen Perforationen beträgt mindestens 19 % und höchstens 23 %, vorzugsweise mindestens 20 % und höchstens 22 % der Flächenerstreckung der Folie. Die Anzahl der in der ersten Folie pro Fläche vorhandenen Öffnungen beträgt mindestens 270 pro cm² und höchstens 290 pro cm². Der Durchmesser der Perforationen, gemessen in der Ebene der Folie, beträgt mindestens 250 µm und höchstens 350 µm. Das Flächengewicht der Folie, gemessen nach EN ISO 2286 - 2, beträgt mindestens 55 g/m² und höchstens 65 g/m².
b) Das zweite Verbandmaterial umfasst einen offenzelligen Polymerschaum, erhältlich durch Umsetzung einer Mischung, umfassend die Komponenten (i) Polyisocyanat, (ii) Polyesterpolyol, (iii) Treibmittel, und (iv) Katalysator. Die Bruchdehnung des Schaums, gemessen gemäß DIN 53571, beträgt 280 % bis 300 %. Der Schaum weist eine Zellzahl (= Anzahl der Poren entlang einer an der Schaumoberfläche in Maschinenrichtung angelegten Geraden pro laufendem cm) von 8 bis 15 pro cm. Die Zellzahl wird bevorzugt mikroskopisch bestimmt. Der Schaum weist eine Rohdichte, gemessen nach DIN EN ISO 845 (Probekörper mit Abmessungen 100 mm x 100 mm x 50 mm, Konditionierung für 24 h im Normklima (23 °C, 50 % rel. Luftfeuchte, 1013 mbar)), zwischen 25,4 und 26,2 kg/m³ und eine Luftdurchlässigkeit, gemessen nach DIN EN ISO 9237 (20 mm Prüfdicke, 20 cm² Prüffläche, 200 Pa Differenzdruck), von 2620 l/(m² sec) bis 2740 l/(m² sec) auf. Ein im Rahmen dieser Ausführungsform besonders gut geeigneter Schaum ist erhältlich aus einer Mischung, umfassend die Komponenten (i) Polyisocyanat, (ii) Polyesterpolyol, (iii) Treibmittel, und (iv) Katalysator, wobei der Polyesterpolyol bevorzugt erhältlich ist durch Umsetzung einer Dicarbonsäure mit 4 bis 8 Kohlenstoffatomen mit einen Dialkohol mit 2 bis 6 Kohlenstoffatomen und/oder bevorzugt ein gewichtsmittleres Molekulargewicht von 500 bis 4000 g/mol aufweist.

Bei Verwendung eines ersten Verbandmaterials und eines zweiten Verbandmaterials mit den unter a) und b) vorstehend genannten Merkmalen kann erreicht werden, dass zum Verschieben des ersten Verbandmaterials gegen das zweite Verbandmaterial im nassen Zustand eine statische Gleitreibungskraft Fₛ von mindestens 3 N erforderlich ist und dass zum Verschieben des ersten Verbandmaterials gegen das zweite Verbandmaterial im trockenen Zustand eine statische Gleitreibungskraft Fₛ von mindestens 6 N erforderlich ist. Bei Verwendung anderer als der hier vorgeschlagenen Materialien kann sich die Notwendigkeit ergeben, die Gleitreibungskraft durch Variation der unter a) und b) vorstehend genannten Parameter zu optimieren. Derartige Versuche können ausgehend von den hier bereits vorgeschlagenen Parameterspannen ohne größeren Aufwand durchgeführt werden.

Sofern in den einschlägigen Normen nicht anders angegeben, werden im Allgemeinen alle Testmethoden bei 23 °C, 50 % rel. Luftfeuchte und 1013 mbar Druck durchgeführt.

Die Erfindung umfasst ein therapeutisches Verfahren zur Behandlung von Wundkavitäten am menschlichen oder tierischen Körper unter Verwendung des vorstehend beschriebenen Verbundes (Wundauflage) aus erstem und zweitem Verbandmaterial, wobei die Behandlung insbesondere unter Anwendung von Unterdruck durchgeführt wird.

Gemäß einer vorteilhaften Ausführungsform der Erfindung wird ein Verfahren zur therapeutischen Behandlung von Wundkavitäten am menschlichen oder tierischen Körper mittels Unterdruck vorgeschlagen, umfassend
i) Bereitstellen einer Unterdruckquelle, eines geeigneten Mittels zum Herstellen einer Unterdruckkommunikation zwischen Unterdruckquelle und Wundraum (beispielsweise eine Unterdruckleitung und ein Unterdruckanschlussstück), eines geeigneten Mittels zum Versiegeln des Wundraums (beispielsweise eine luftdichte Abdeckfolie) sowie optional eines Behälters für die abgesaugten Wundfluide,
ii) Bereitstellen eines ersten Verbandmaterials als Wundkontaktschicht, umfassend eine flexible, perforierte Folie mit einer ersten und einer zweiten Seite, wobei die in der Folie vorhandenen Perforationen derart in die Folie eingebracht werden, dass die Perforationsränder von der zweiten Seite der Folie abstehen, so dass auf der zweiten Seite der Folie dreidimensional ausgestaltete Strukturen vorhanden sind und wobei die erste Seite zum Inkontaktbringen mit einem Wundgrund, insbesondere mit der inneren Oberfläche einer Wundröhre, vorgesehen ist,
iii) Bereitstellen eines zweiten Verbandmaterials zum Einbringen in eine Wundröhre, umfassend einen porösen Polymerschaum, wobei es sich bei dem Polymerschaum um einen offenzelligen Polymerschaum handelt, welcher an oder nahe seiner Oberfläche Stege umfasst und/oder welcher auf seiner Oberfläche zur Oberfläche hin offene Hohlräume umfasst, und wobei die Stege und/oder Hohlräume dreidimensional ausgestaltete Strukturen bilden,
iv) Aufbringen der zweiten Seite der Folie auf die Oberfläche des Polymerschaums unmittelbar vor der Unterdruckbehandlung, wobei der Polymerschaum teilweise oder vollständig durch die Folie umhüllt ist, und wobei die auf den Oberflächen von Polymerschaum und Folie vorhandenen dreidimensional ausgestalteten Strukturen nach dem Inkontaktbringen von Polymerschaum und Folie eine haftende Verbindung eingehen,
v) Einbringen des Verbundes aus erstem und zweitem Verbandmaterial in eine Wundkavität, wobei aufgrund der haftenden Verbindung zwischen den Oberflächen von Polymerschaum und Folie ein Ablösen der Folie vom Polymerschaum während des Einbringens weitestgehend vermieden werden kann,
vi) Herstellen einer luftdichten Abdeckung des Wundraumes und Anschließen einer Unterdruckquelle,
vii) und Durchführen der Unterdrucktherapie.

Durch die mit Schritt iv) hergestellte haftende Verbindung von Polymerschaum und Folie soll ein Ablösen der Folie vom Polymerschaum und/oder ein Verschieben von Polymerschaum und Folie während des auf Schritt iv) folgenden Einbringens des Verbundes aus erstem und zweitem Verbandmaterial in eine Wundkavität weitestgehend vermieden werden. Nach dem Aufbringen der zweiten Seite der Folie auf die Oberfläche des Polymerschaums muss zum Verschieben der Folie im trockenen Zustand gegen den Polymerschaum im trockenen Zustand eine statische Gleitreibungskraft Fₛ von mindestens 6 N, gemessen nach DIN EN ISO 8235, erforderlich sein. Hierdurch wird eine besonders sichere Haftverbindung von Polymerschaum und Folie gewährleistet.

Optional können nach Schritt v) und vor der Abdichtung des Wundraums ein oder mehrere weiterer Verbandlagen aufgebracht werden, beispielsweise eine weitere Schaumlage.

Üblicherweise erfolgt vor Beginn der Unterdrucktherapie, also noch vor Schritt i), zunächst eine Exploration der Wunde, welche gegebenenfalls eine Sondierung der Kavität umfasst. Danach kann der Anwender sowohl die perforierte Folie als auch den porösen Polymerschaumstoff an die Abmessung der Wunde anpassen, beispielsweise durch Zuschneiden. Optional umfasst das vorstehend beschriebene Verfahren somit in Schritt ii) das Anpassen der Folie an die Abmessung der Wunde und/oder in Schritt iii) das Anpassen des Polymerschaumstoffs an die Abmessung der Wunde.

Das Aufbringen der zweiten Seite der Folie auf die Oberfläche des Polymerschaums (Schritt iv) erfolgt unmittelbar vor dem Anlegen der Wundauflage. Der Begriff "Aufbringen" umfasst im hier vorliegenden Zusammenhang ein teilweises oder vollständiges Umhüllen des Schaumstoffs durch die Folie. Die Folie kann hierbei optional in mehreren Windungen um das zweite Verbandmaterial gewickelt werden, so dass der Schaumstoff teilweise oder vollständig von mehr als einer Lage Folie umhüllt ist. Idealerweise sollten nur ein bis drei Lagen der Folie übereinander gebracht werden, da ansonsten die Durchlässigkeit der Hülle für Wundexsudat in unerwünschter Weise nachlässt.

Gemäß einer für die Behandlungspraxis bevorzugten Ausführungsform wird nur eine Lage der flexiblen Folie als Hülle auf den Schaumstoff aufgebracht.

Im Falle eines zylinderförmig ausgestalteten Schaumstoffblockes kann optional nur der Mantel des Zylinders, welcher bei der Therapie die wesentliche Kontaktfläche mit dem Wundgrund darstellt, mit der ersten Folie abgedeckt werden, während die Querschnittsflächen des Zylinders unbedeckt bleiben. Wird die bei der Anwendung in Richtung auf die Unterdruckquelle hin zeigende Querschnittsfläche nicht mit der Folie abgedeckt, so kann sich hierbei in vorteilhafter Weise eine verbesserte Weiterleitung von Wundexsudat zur Unterdruckquelle ergeben.

Erfindungsgemäß muss mindestens 75 % der Oberfläche des zweiten Verbandmaterials durch die Folie abgedeckt sein, da ansonsten eine Verklebung und/oder Verwachsung von Granulationsgewebe mit dem Schaumstoff zu befürchten ist. Vorzugweise wird mindestens 85 % der Oberfläche des zweiten Verbandmaterials durch die Folie abgedeckt, besonders bevorzugt mindestens 95 %. Gemäß einer weiteren besonders bevorzugten Ausführungsform wird die gesamte Oberfläche des zweiten Verbandmaterials durch die Folie abgedeckt.

Überraschenderweise wurde gefunden, dass die Folie nicht notwendigerweise glatt auf den Polymerschaum aufgebracht werden muss. Stattdessen kann die Oberfläche der den Polymerschaum umhüllenden Folie durchaus Einfalzungen oder Verwerfungen aufweisen. Einfalzungen oder Verwerfungen können dazu führen, dass die Folie partiell mehrlagig auf dem Schaum vorhanden ist. Aufgrund der bereits erwähnten Kapillarwirkung kann auch in den Bereichen, in denen Einfalzungen oder Verwerfungen vorhanden sind, eine ausreichende Ableitung von Wundexsudat erfolgen.

In vorteilhafter Weise kann nach Beendigung der Unterdrucktherapie der Polymerschaum und die Folie in einem Schritt entfernt werden. Dies ist insbesondere dann möglich, wenn die mit Schritt iv) hergestellte haftende Verbindung von Polymerschaum und Folie auch in einem nassen Zustand der vorgenannten Komponenten vorhanden ist. Hierzu muss die zum Verschieben der Folie im nassen Zustand gegen den Polymerschaum im nassen Zustand erforderliche statische Gleitreibungskraft Fₛ mindestens 3 N, gemessen nach DIN EN ISO 8235, betragen. Hierdurch wird eine besonders sichere Haftverbindung von Polymerschaum und Folie während der gesamten Therapie gewährleistet.

Gemäß einer bevorzugten Ausführungsform umfasst das Verbandset einen Polymerschaum, in dessen Inneren ein Drainageschlauch oder ein anderes geeignetes Leitungsmittel eingebettet vorliegt. Der Schlauch kann beispielsweise bereits herstellerseitig in den Schaumstoff eingebracht werden. Es ist auch möglich, dass der Anwender vor der Applikation der Wundauflage in den Schaumstoff ein durchgehendes oder blind endendes Loch einbringt, in welches der Drainageschlauch dann eingeführt wird. Eine im Inneren des Schaumstoffblocks vorhandene Drainageleitung kann gegebenenfalls die Ableitung von Wundexsudat aus der Kavität und/oder das Offenhalten der Kavität während der Unterdruckapplikation unterstützen. Eine derartige Ausführungsform könnte beispielsweise bei der Unterdruckbehandlung röhrenförmiger Kavitäten, insbesondere von durchgehenden Tunnelwunden, mit einem Durchmesser von mehr als zwei Zentimetern eingesetzt werden.

Gemäß einer weiteren vorteilhaften Ausführungsform des Verbandsets kann optional am ersten Verbandmaterial und/oder am zweiten Verbandmaterial mindestens eine zusätzliche Komponente vorhanden sein, welche die haftende Verbindung zwischen dem ersten Verbandmaterial und dem zweiten Verbandmaterial weiter verstärken kann. Bei der zusätzlichen Komponente kann es sich beispielsweise um eine adhäsive Beschichtung, welche an der zweiten Oberfläche des ersten Verbandmaterials und/ oder an der Oberfläche des zweiten Verbandmaterials vorhanden ist, handeln. Vorzugweise ist die adhäsive Beschichtung auf die zweite Oberfläche des ersten Verbandmaterials aufgebracht, da eine Beschichtung der Oberfläche des zweiten Verbandmaterials bei der Größenanpassung des Schaumstoffs verlorengehen kann. Ein weiterer Vorzug einer auf die zweite Oberfläche des ersten Verbandmaterials aufgebrachten adhäsiven Beschichtung besteht darin, dass die Folienbereiche, welche beim Umwickeln des Schaumstoffs aufeinander zu liegen kommen, dann gleichfalls aufeinander haften.

Im Falle komplexer Wunden können gleichzeitig nach außen hin offene Wundbereiche, welche keine Kavitäten aufweisen, und Bereiche mit einer oder mehreren Wundkavitäten vorhanden sein. So können, beispielsweise infolge von inflammatorischen Prozessen, Wundröhren am Rand eines zentralen, nach außen hin offenen Wundbereiches, welcher einen ausgedehnten Hautdefekt aufweist, vorhanden sein. Hierzu wird im Zusammenhang mit der vorliegenden Erfindung vorgeschlagen, die eine oder die mehreren Kavitäten mit der erfindungsgemäßen Wundauflage zu versorgen und die offenen Bereiche mit einem porösen Verbandmaterial, insbesondere mit einem offenzelligen Polymerschaum auszukleiden. Es ist jedoch auch denkbar, die nach außen hin offenen Wundbereiche gleichfalls mit der erfindungsgemäßen Wundauflage zu bedecken.

Häufig ist auch die Behandlung von Wunden erforderlich, welche nur einen geringen Hautdefekt aufweisen, jedoch mehrere Wundkavitäten unterschiedlicher Größe umfassen. Im Falle einer derartigen Wunde kann eine vergleichsweise große Wundtasche vorliegen, beispielsweise im Zentrum der Wunde, an die sich eine oder mehrere vergleichsweise enge Wundröhren anschließen. Im Zusammenhang mit der vorliegenden Erfindung kann es sich in einer solchen Situation als vorteilhaft erweisen, die erfindungsgemäßen Wundauflage lediglich in die engen Wundröhren einzubringen, während die eine größere Abmessung aufweisende zentrale Tasche mit einem porösen Verbandmaterial, insbesondere mit einem offenzelligen Polymerschaum ausgekleidet wird.

Gemäß einem weiteren Aspekt der Erfindung wird ein Erzeugnis, zur Anwendung in der therapeutischen Behandlung von Wundkavitäten am menschlichen oder tierischen Körper mittels Unterdruck vorgeschlagen. Das Erzeugnis wird hierbei also ausdrücklich im Zusammenhang mit seiner speziellen medizinischen Verwendung beziehungsweise Indikation, nämlich der Behandlung von Wundkavitäten, beansprucht. Das Erzeugnis umfasst i) ein separat bereit gestelltes erstes Verbandmaterial als Wundkontaktschicht, umfassend eine flexible, perforierte Folie mit einer ersten und einer zweiten Seite, wobei die in der Folie vorhandenen Perforationen derart beschaffen sind, dass die Perforationsränder von der zweiten Seite der Folie abstehen, so dass auf der zweiten Seite der Folie dreidimensional ausgestaltete Strukturen vorhanden sind und wobei die erste Seite zum Inkontaktbringen mit einem Wundgrund, insbesondere mit der inneren Oberfläche einer Wundröhre, vorgesehen ist, und ii) ein separat bereit gestelltes zweites Verbandmaterial zum Einbringen in eine Wundröhre, umfassend einen porösen Polymerschaum, wobei es sich bei dem Polymerschaum um einen offenzelligen Polymerschaum handelt, welcher an oder nahe seiner Oberfläche Stege umfasst und/oder welcher auf seiner Oberfläche zur Oberfläche hin offene Hohlräume umfasst, und wobei die Stege und/oder Hohlräume dreidimensional ausgestaltete Strukturen bilden. Das Erzeugnis zeichnet sich weiter dadurch aus, dass die auf der zweiten Seite des ersten Verbandmaterials vorhandenen Strukturen mit den auf der Oberfläche des zweiten Verbandmaterials vorhandenen Strukturen eine haftende Verbindung eingehen können, und dass die zweite Seite der Folie zum teilweisen oder vollständigen Umhüllen der Oberfläche des zweiten Verbandmaterials unmittelbar vor der Unterdruckbehandlung vorgesehen ist, so dass nach dem Einbringen des Verbundes aus erstem und zweitem Verbandmaterial in eine Wundkavität aufgrund der haftenden Verbindung zwischen dem ersten Verbandmaterial und dem zweiten Verbandmaterial ein Verschieben des ersten Verbandmaterials gegenüber dem zweiten Verbandmaterial während der Therapie weitestgehend vermieden werden kann und/oder das gleichzeitige Entfernen von dem ersten Verbandmaterial und dem zweitem Verbandmaterial erleichtert wird.

Ein weiterer Aspekt im Rahmen der Erfindung bezieht sich auf ein Erzeugnis, zur Vermeidung von Komplikationen bei der therapeutischen Behandlung von Wundkavitäten am menschlichen oder tierischen Körper mittels Unterdruck. Bei der Komplikation handelt es sich um das Zurückbleiben von Schaumstoffpartikeln im Wundbereich, insbesondere in einer Wundkavität. Das Erzeugnis wird hierbei also ausdrücklich im Zusammenhang mit seiner speziellen medizinischen Verwendung beziehungsweise Indikation, nämlich der Vermeidung einer häufigen und schweren Komplikation bei der Behandlung von Wundkavitäten, beansprucht. Bei dem Erzeugnis handelt es sich um das vorstehend beschriebene Erzeugnis zur Anwendung in der therapeutischen Behandlung von Wundkavitäten am menschlichen oder tierischen Körper mittels Unterdruck.

Des Weiteren bezieht sich die Erfindung auf ein Erzeugnis, zur Vereinfachung der therapeutischen Behandlung von Wundkavitäten am menschlichen oder tierischen Körper mittels Unterdruck. Das Erzeugnis wird hierbei also ausdrücklich im Zusammenhang mit seiner speziellen medizinischen Verwendung, nämlich der Vereinfachung der Behandlung von Wundkavitäten, beansprucht. Bei dem Erzeugnis handelt es sich um das vorstehend beschriebene Erzeugnis zur Anwendung in der therapeutischen Behandlung von Wundkavitäten am menschlichen oder tierischen Körper mittels Unterdruck.

Weiterhin ist die Erfindung auf eine flexible, perforierte Folie als Wundkontaktschicht, zur Anwendung bei der Behandlung von Wundkavitäten mittels Unterdruck, gerichtet. Die Folie wird hierbei ausdrücklich im Zusammenhang mit ihrer speziellen medizinischen Verwendung, nämlich der Verwendung als Wundkontaktschicht bei der Unterdruckbehandlung von Wundkavitäten, beansprucht. Die Folie weist hierbei eine weitgehend glatte erste Seite, welche zum Inkontaktbringen mit einem Wundgrund, insbesondere mit der inneren Oberfläche einer Wundröhre, vorgesehen ist und eine raue zweite Seite auf. Die in der Folie vorhandenen Perforationen sind derart beschaffen, dass die Perforationsränder von der zweiten Seite der Folie abstehen, so dass auf der zweiten Seite der Folie dreidimensional ausgestaltete Strukturen vorhanden sind. Die dreidimensional ausgestalteten Strukturen können mit dreidimensional ausgestalteten Strukturen, welche auf einer weiteren Verbandlage vorhanden sind, eine haftende Verbindung eingehen. Bei der weiteren Verbandlage kann es sich insbesondere um einen offenzelligen Polymerschaum handeln, welcher als Wundfüller geeignet ist.

Im Rahmen der Erfindung wird gleichfalls ein gebrauchsfertiges Verbandset beansprucht, welches zur Anwendung in der therapeutischen Behandlung von Wundkavitäten am menschlichen oder tierischen Körper mittels Unterdruck geeignet ist, umfassend einen Verbund (Wundauflage) nach einem der vorstehend beschriebenen Ausführungsformen, wobei das erste und das zweite Verbandmaterial jeweils steril vorliegen, sowie optional eine luftdichte Abdeckfolie zum Verschließen des Wundraums und/oder ein Unterdruckanschlussstück. Die einzelnen Komponenten sollten hierbei jeweils separat steril abgepackt vorliegen, wobei die Gesamtheit der Komponenten wiederum in einer einzigen Umverpackung untergebracht sein könnte. Idealerweise erfolgt die Sterilisation nach dem Verpacken der Komponenten in der Umverpackung, beispielsweise unter Verwendung von Ethylenoxid.

Gleichfalls von der Erfindung umfasst ist eine Vorrichtung, geeignet zur Verwendung bei der Behandlung von Wundkavitäten mittels Unterdruck, umfassend eine Wundauflage nach einem der vorstehend beschriebenen Ausführungsformen, weiterhin umfassend eine luftdichte Abdeckfolie, ein Unterdruckanschlussstück, eine Unterdruckleitung, eine Unterdruckquelle sowie optional einen Behälter für die abgesaugten Wundfluide.

### Anwendungsbeispiel I

Schusswunde am Oberarm (Durchschuss) mit durchgehender röhrenförmiger Kavität.

Nach Exploration der Wunde erfolgt zunächst ein Debridement. Zur Versorgung der röhrenartig ausgebildeten Wundkavität wird vom behandelnden Arzt ein an die Größe der Wundröhre angepasster quaderförmiger Schaumstoffblock (mit den Seiten a; b; c, wobei die Längen von a beispielsweise 2 cm, von b beispielsweise 2 cm und von c beispielsweise 7 cm betragen) mit einer mikroperforierten Polyethylen-Folie (Breite der Folienbahn 3 cm) straff umwickelt. Es werden etwa drei Windungen versetzt aufgelegt, so dass im Überlappungsbereich zwei Folienlagen und außerhalb des Überlappungsbereichs jeweils eine Folienlage auf dem Schaum vorhanden ist. Die beiden Seitenflächen (a; b und gegenüberliegend a'; b') werden nicht mit Folie bedeckt, so dass im beispielhaft genannten Fall ca. 88 % der Schaumstoffoberfläche mit einer Folie überzogen vorliegen. Ein geeigneter Schaumstoff ist beispielsweise das kommerziell erhältliche Produkt VivanoMed^{®} Foam (Paul Hartmann AG, Deutschland), ein offenzelliger Polymerschaum aus Polyester-Polyurethan. Die Polyethylenfolie umfasst vorzugsweise etwa 270 bis 290 Perforationen pro cm² Folienfläche mit einem Durchmesser von jeweils 0,3 mm. Die Perforationen sind vorzugsweise derart in der Folie vorhanden, dass die Perforationsränder von der Oberfläche der Folie abstehen. Somit ist auf einer Oberfläche der Folie eine kraterförmige, dreidimensional ausgestaltete Struktur vorhanden, während die andere gegenüberliegende Oberfläche der Folie weitgehend glatt ausgebildet ist. Die offene Fläche der Folie beträgt vorzugsweise 20 % bis 22 %. Die Folie wird beim Umwickeln des Schaumstoffs mit ihrer rauen Seite (abstehende Perforationsränder) auf den Schaum gewickelt. Die Folie haftet hierbei auf dem Schaumstoff. Die derart präparierte Wundauflage, welche nach dem Umwickeln eine insgesamt röhrenförmige Gestalt aufweist, wird durch die auf der Ausschussstelle vorhandene Öffnung in den Wundtunnel eingebracht. Die Enden der Wundauflage sollten sich vorzugsweise auf beiden Seiten des Wundkanals ca. 1 bis 2 cm unter der Ebene der unversehrten Wundumgebung befinden. Auf die Enden der Wundauflage wird jeweils ein zurechtgeschnittener Schaumstoffblock (VivanoMed^{®} Foam) so aufgebracht, dass die Einschussstelle und die üblicherweise größere Ausschussstelle vollständig bis zur Ebene der unversehrten Wundumgebung mit dem Schaum ausgefüllt werden. Auf den Schaumstoff wird zur luftdichten Abdeckung der Wunde sowohl an der Einschussstelle als auch an der Ausschussstelle ein transparenter selbstklebender Filmverband (Hydrofilm^{®}, Paul Hartmann AG, Deutschland) aufgebracht und an der unversehrten, die die Wunde umgebenden Haut fixiert. Nachfolgend wird in einen der auf beiden Seiten des Wundkanals vorhandenen Unterdruckverbände ein Loch in den Filmverband geschnitten. Das Loch wird vorzugsweise in den transparenten Filmverband eingebracht, welcher sich über der Ausschussstelle befindet. Über dem Loch wird ein Unterdruckanschlussstück (beispielsweise ein VivanoTec^{®} Port, Paul Hartmann AG, Deutschland) dichtend befestigt. Das Unterdruckanschlussstück kann sodann über eine Sekretleitung mit einer Unterdrucktherapieeinheit (VivanoTec^{®}, Paul Hartmann AG, Deutschland) verbunden werden. Die Unterdrucktherapieeinheit umfasst vorzugsweise einen auswechselbaren Sekretbehälter, beispielsweise mit einem Fassungsvermögen von 850 ml. Anschließend wird ein konstanter Unterdruck von 125 mm Hg für eine Dauer zwei Tagen angelegt. Nach vorzugsweise zwei Tagen wird der Verband gewechselt und die Wunde beurteilt. Aufgrund der Haftung zwischen Folie und Schaumstoff kann der umhüllte Schaumkörper, der in den Wundkanal eingebracht wurde, einfach und in einem Stück entnommen werden.

Die Behandlung wird, wie oben beschrieben, solange wiederholt, bis ausreichend Granulationsgewebe vorhanden und die Wundröhre zugewachsen ist. Vor dem Anlegen eines neuen Unterdruckverbandes erfolgt dabei normalerweise eine Wiederholung des Debridements.

Nachdem sich ausreichend Granulationsgewebe gebildet hat und der Wundtunnel geschlossen ist, kann der Patient beispielsweise für eine Spalthauttransplantation vorbereitet oder mit konventionellen Wundprodukten weiter versorgt werden. Bei der Spalthauttransplantation wird eine dünne Hautschicht von einer anderen Körperstelle des Patienten entnommen. Im Falle einer Spalthauttransplantation kann die Wunde in vorteilhafter Weise erneut mittels Unterdrucktherapie versorgt werden. Verfahren zur Behandlung einer derartigen Transplantationswunde mittels Unterdruck sind aus dem Stand der Technik bekannt.

### Anwendungsbeispiel II

Komplexe Dekubitalwunde mit einer zentralen, großflächigen und nach außen hin offenen Wundhöhle und einer seitlich davon abgehenden röhrenförmigen Wundtasche, wobei die Wundtasche blind endet.

Nach Exploration der Wunde erfolgt wie bei Anwendungsbeispiel I zunächst ein Debridement. Die Abmessungen des subkutanen Wundkanals werden durch Sondierung abgeschätzt. Zur Versorgung des röhrenartig ausgebildeten subkutanen Wundbereichs wird vom behandelnden Arzt analog Anwendungsbeispiel I ein an die Größe der Wundröhre angepasster quaderförmiger Schaumstoffblock geeigneter Größe mit einer mikroperforierten Polyethylen-Folie straff umwickelt. Die derart präparierte Wundauflage, welche nach dem Umwickeln eine insgesamt röhrenförmige Gestalt aufweist, wird vollständig in den blind endenden Wundkanal eingebracht. Aufgrund der Haftung zwischen Folie und Schaumstoff bleibt die unmittelbar vor der Behandlung aufgebrachte Umhüllung des Schaumstoffs während des Einbringens der Wundauflage in die Kavität intakt. Der zentrale, offene Bereich der Wunde wird mit einem durch Zuschneiden an die Wundform angepassten Schaumstoffblock (VivanoMed^{®} Foam) bedeckt. Vorzugsweise kann der Wundgrund im offenen Bereich der Wunde vor der Applikation des Schaumstoffs mit einer geeigneten Wundkontaktschicht (beispielsweise Atrauman Ag^{®}, Paul Hartmann AG, Deutschland) bedeckt werden. Der gesamte Wundbereich wird zur luftdichten Versiegelung der Wunde mit einem transparenten, selbstklebenden Filmverband (Hydrofilm^{®}, Paul Hartmann AG, Deutschland) abgedeckt. Der Filmverband wird an der intakten, die Wunde umgebenden Haut fixiert. In den Filmverband wird ein Loch eingebracht. Über dem Loch wird wie bei Anwendungsbeispiel I ein Unterdruckanschlussstück (beispielsweise ein VivanoTec^{®} Port, Paul Hartmann AG, Deutschland) dichtend befestigt. Das Unterdruckanschlussstück kann sodann über eine Sekretleitung mit einer Unterdrucktherapieeinheit (VivanoTec^{®}, Paul Hartmann AG, Deutschland) verbunden werden. Anschließend wird ein konstanter oder variabler Unterdruck, beispielsweise im Bereich von 80 bis 125 mm Hg, für eine Dauer von vorzugsweise ein bis drei Tagen angelegt. Da die Haftung zwischen Folie und Schaumstoff auch während der Therapie erhalten bleibt, kann der umhüllte Schaumkörper, der in den subkutanen Wundkanal eingebracht wurde, bei dem auf die Unterdrucktherapie folgenden Verbandwechsel einfach und in einem Stück entnommen werden. Die Umhüllung verhindert weitestgehend, dass Schaumstoffpartikel in der Wundkavität zurückbleiben können.

Die Behandlung kann solange wiederholt werden, bis ausreichend Granulationsgewebe im Wundkanal vorhanden und die Wundröhre zugewachsen ist. Vor dem Anlegen eines neuen Unterdruckverbandes erfolgt normalerweise eine Wiederholung des Debridements. Zur Behandlung des offenen Wundbereichs kann die Unterdrucktherapie unter Verwendung üblicher, aus dem Stand der Technik bekannter Verbandkomponenten bis zur Heilung der Wunde fortgesetzt werden.

### Bestimmung der Gleitreibungskraft Fₛ

Die Bestimmung der statischen Gleitreibungskraft Fₛ einer Folien-Oberfläche gegen eine Schaumstoff-Oberfläche erfolgte analog DIN EN ISO 8295 (Ausgabe Oktober 2004) unter Verwendung des in der Norm beschriebenen Prüfgerätes (Zugprüfmaschine der Firma Zwick, Deutschland), wobei ein waagrechter Prüftisch eingesetzt wurde. Es wurden jeweils drei Proben gemessen und der Mittelwert aus den drei Messungen berechnet. Zur Messung der Proben im trockenen Zustand erfolgte vor der Prüfung eine Konditionierung für mindestens 16 h im Normklima bei 23°C, 50 % relative Luftfeuchte, 1013 mbar. Zur Messung der Proben im nassen Zustand wurden die Proben (Schaumstoff und Folie) vollständig im Wasser eingetaucht. Danach wurden die Proben zum Abtropfen 30 s vertikal gehalten und in das Prüfgerät eingespannt. Die Schaumstofflage (Abmessung 150 x 300 mm) wurde an dem Prüftisch befestigt, während die Folienlage (Abmessung 65 x 200 mm) in den Schlitten eingespannt wurde. Die Zeitspanne zwischen dem Abtropfen und dem Beginn der Messung betrug maximal 2 min. Die mit der Folie bespannte quadratische Kontaktgrundfläche des Schlittens betrug 40 cm². Der Schlitten wurde über den unbeweglichen Prüftisch gezogen. Die Prüfgeschwindigkeit betrug 100 mm/min. Das Gewicht des Zugschlittens einschließlich des Reibklotzes betrug 200 g. Die Vorspannung zwischen Schlitten und Kraftmessgerät betrug 0,2 N.

Beispiel: Bestimmung der Gleitreibungskraft einer ersten Verbandlage gegen eine zweite Verbandlage an einem Prüfgerät.

Die Bestimmung der statischen Gleitreibungskraft Fₛ wurde wie vorstehend beschrieben analog DIN EN ISO 8295 durchgeführt.

Probenpaar 1 - Offenzelliger Schaumstoff gegen eine Schlitzfolie, welche eine weitgehend glatte Oberfläche aufweist.

Schaumstoff: Hydrophober Polyester-Polyurethan-Schaumstoff. Rohdichte gemäß ISO 845 25,8 kg/m³, Stauchhärte gemäß DIN EN ISO 3386-1 3,9 kPa, Zugfestigkeit gemäß DIN 53571 A 170 kPa, Bruchdehnung gemäß DIN 53571 A 290 %, Zellzahl (mikroskopisch bestimmt an einer an der Oberfläche des Schaumes angelegten geraden Linie) 11/cm, Luftdurchlässigkeit gemäß DIN EN ISO 9237 2680l/m²s. Ein derartiger, als Wundauflage verwendbarer Schaumstoff, ist unter der Bezeichnung "VivanoMed^{®}-Schaum" kommerziell erhältlich (Paul Hartmann AG, Deutschland).

Folie: Glatte, transparente Polyurethan-Folie mit Schlitzen. In die Folie wurden pro 100 cm² Folienoberfläche ca. 20 über die Fläche der Folie verteilte Schlitze mit einer Länge von jeweils 4,5 mm eingebracht. Die Ausrichtung der Schlitze erfolgte parallel zur Maschinenrichtung (MD) der Folie. Bei einem Einsatz einer derartigen Folie als Wundkontaktschicht beziehungsweise als Organschutzschicht dienen die Schlitze der Durchleitung von Wundexsudat. Die Verwendung geschlitzter Folien als WundkontaktSchicht, insbesondere im Zusammenhang mit der Unterdruck-Therapie, ist aus dem Stand der Technik bekannt.

Die Folie wurde derart in den Messschlitten eingespannt, dass die Bewegung des Schlittens entlang der Maschinenrichtung der Folie erfolgte.

Probenpaar 2 - Offenzelliger Schaumstoff gegen eine Schlitzfolie, welche auf einer Oberfläche eine dreidimensional ausgestaltete kraterförmige Struktur umfasst.

Schaumstoff: Hydrophober Polyester-Polyurethan-Schaumstoff. Rohdichte gemäß ISO 845 25,8 kg/m³, Stauchhärte gemäß DIN EN ISO 3386-1 3,9 kPa, Zugfestigkeit gemäß DIN 53571 A 170 kPa, Bruchdehnung gemäß DIN 53571A 290 %, Zellzahl (mikroskopisch bestimmt an einer an der Oberfläche des Schaumes angelegten geraden Linie) 11/cm, Luftdurchlässigkeit gemäß DIN EN ISO 9237 2680l/m²s. Ein derartiger, als Wundauflage verwendbarer Schaumstoff, ist unter der Bezeichnung VivanoMed^{®} Foam kommerziell erhältlich (Paul Hartmann AG, Deutschland).

Folie: Einseitig raue transparente Polyethylen-Folie mit Perforationen. Die Folie umfasst etwa 280 Perforationen pro cm² Folienfläche mit einem Durchmesser von jeweils 0,3 mm. Die Perforationen wurden derart in die Folie eingebracht, dass die Perforationsränder von der zweiten Oberfläche der Folie abstehen, so dass auf der zweiten Oberfläche eine kraterförmige, dreidimensional ausgestaltete Struktur vorhanden ist. Die erste Oberfläche der Folie hingegen ist weitgehend glatt ausgebildet. Die offene Fläche der Folie beträgt 21 %. Die Folie wurde derart in den Messschlitten eingespannt, dass die Bewegung des Schlittens entlang der Maschinenrichtung (MD) der Folie erfolgte. Die raue Seite der Folie (zweite Oberfläche) wurde zum Prüftisch hin ausgerichtet, so dass die raue Seite mit dem Schaumstoff in Kontakt stand.

Die zwischen den Materiallagen (Probenpaare) vorhandene statische Gleitreibungskraft wurde jeweils bei trockenen und nassen Proben mittels der vorstehend angegebenen Messmethode bestimmt. Im Folgenden sind die Mittelwerte der gemessenen Gleitreibungskraft Fₛ aus jeweils drei Messungen angegeben.
- Probenpaar 1 - trocken: 0,98 N
- Probenpaar 1 - nass: 0,72 N

- Probenpaar 2 - trocken: 10,08 N
- Probenpaar 2 - nass: 5,86 N

### Bezugszeichen

- 1: Unterdruckquelle
- 2: Sekretbehälter (Kanister für Wundexsudat)
- 3: Wundgrund
- 5: Wundrand
- 6: luftdichte Abdeckfolie zum Verschließen des Wundraums
- 7: Unterdruck-Anschlussmittel (Port)
- 8: Unterdruckleitung
- 9: Öffnung in Abdeckfilm
- 10: Von einer perforierten Folie teilweise umhüllter Schaumstoff
- 11,21,31: perforierte, flexible Folie (Wundkontaktschicht)
- 12, 22, 32, 35: offenzelliger Polymerschaum
- 13: zur Oberfläche hin offener Hohlraum in dem offenzelligen Polymerschaum
- 14: an oder nahe der ersten Oberfläche des offenzelligen Polymerschaumes vorhandener Schaumstoff-Steg
- 15: Perforation in der flexiblen Folie
- 16: Perforationsrand in der flexiblen Folie. Der Perforationsrand steht von der zweiten Oberfläche der Folie ab, so dass auf der zweiten Oberfläche der Folie eine dreidimensional ausgestaltete Struktur gebildet wird
- 20, 30: an einer Wunde angebrachte Vorrichtung zur therapeutischen Behandlung von Wundkavitäten mittels Unterdruck
- 25: erste Wundstelle
- 26: zweite Wundstelle
- 24: durchgehende Wundröhre (Tunnelwunde)
- 33: zentrale, nach außen hin offenen Wundhöhle
- 34: blind endende Wundröhre (Wundtasche)

### Figuren

Nachstehend wird das erfindungsgemäße Verbandset beziehungsweise die Verwendung des Verbandsets im Rahmen einer Vorrichtung zur Unterdrucktherapie von Wunden anhand schematischer Zeichnungen (nicht maßstabsgetreu) näher erläutert. Die Erfindung soll jedoch nicht auf die in den Zeichnungen oder in der Beschreibung der Zeichnung dargestellten Ausgestaltungen reduziert verstanden werden. Vielmehr umfasst die Erfindung auch Kombinationen der Einzelmerkmale der alternativen Formen.

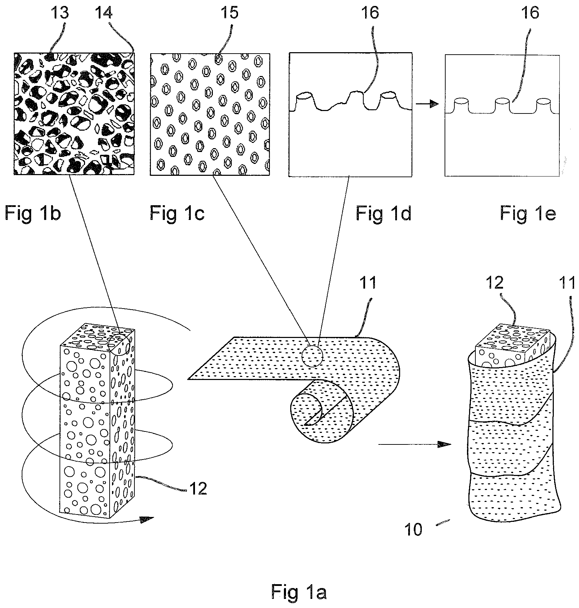

Fig. 1 a zeigt in schematischer Darstellung eine bevorzugte Ausführungsform der Erfindung.
Fig. 1b zeigt eine Zeichnung nach einer mikroskopischen Aufnahme (REM) eines als zweites Verbandmaterial geeigneten offenzelligen Polymerschaums in der Aufsicht. Die Figur zeigt somit ein Oberflächendetail (Originalgröße 6 mm x 6 mm) von Verbandmaterial 12 aus Fig 1a.
Fig. 1 c/d zeigen Zeichnungen nach mikroskopischen Aufnahmen (Auflichtmikroskop) einer als erstes Verbandmaterial geeigneten perforierten Folie in der Aufsicht auf die zweite Seite (Fig. 1c; Originalgröße ca. 23 mm x 23 mm) und von der Seite (Fig. 1d; Originalgröße ca. 9 mm x 9 mm). Die Figuren zeigen somit ein auf der zweiten Seite von Lage 11 aus Figur 1 a vorhandenes Oberflächendetail.
Figur 1e zeigt das Oberflächendetail aus Figur 1d in schematischer Darstellung.
Fig. 2 zeigt einen an eine tunnelartig ausgebildete Wundkavität angelegten Unterdruckverband in schematischer Darstellung.
Fig. 3 zeigt einen an eine Wunde angelegten Unterdruckverband in schematischer Darstellung, wobei die Wunde eine blind endende Kavität umfasst.

Die Figur 1 a stellt beispielhaft eine bevorzugte Ausführungsform des erfindungsgemäßen Verbandsets (10) dar, welches ein vor der Anwendung separat bereit gestelltes, erstes Verbandmaterial (11) als Wundkontaktschicht und ein im vorliegenden Beispiels quaderförmig ausgebildetes zweites Verbandmaterial (12, 22) als Wundfüller umfasst, wobei das erste und das zweite Verbandmaterial aufgrund seiner an den Oberflächen vorhandenen dreidimensional ausgebildeten Strukturen aneinander haften kann. Der Wundfüller (zweites Verbandmaterial 12, 22) kann somit ohne zusätzliche Hilfsmittel unmittelbar vor der Anwendung durch den Arzt mit einer Wundkontaktschicht (erstes Verbandmaterial 11, 21) umhüllt werden, wobei der Verbund während der gesamten Dauer der Therapie einschließlich dem Entfernen der Wundauflage nach der Behandlung erhalten bleibt. Auf der linken Seite von Figur 1 a sind das separat bereitgestellte erste und zweite Verbandmaterial dargestellt, während rechts ein durch die Folie (erstes Verbandmaterial) umhüllter Schaumstoff (zweites Verbandmaterial) abgebildet ist. Die Folie (11) wurde hierzu in etwa drei Windungen um den quaderförmig ausgebildeten Schaumstoffblock (12) gewickelt. Aufgrund der aneinander haftenden Oberflächen von Folie (11) und Schaumstoff (12) ist eine weitere Fixierung der Folie (11) auf dem Schaumstoff (12) nicht erforderlich. Die obere und die untere (in Figur 1a nicht sichtbar) Seitenfläche des Schaumstoffblocks (12) wurde bei dem in Figur 1a dargestellten Beispiel nicht mit Folie bedeckt. Gleichwohl kann der Schaumstoffblock auch vollständig durch die Folie (11) umhüllt werden (nicht dargestellt). Die als Umhüllung der Schaumstoffs (12) und Wundkontaktschicht dienende Folie (11) kann auf beliebige Weise auf den Schaumstoff aufgebracht werden, beispielsweise durch Umwickeln oder durch Auflegen einzeln zurechtgeschnittener Folienabschnitte. Vorzugsweise wird eine einzige streifenförmig ausgebildete Folienbahn aufgetragen. Wesentlich ist, dass mindestens 75 % der Oberfläche der Schaumstoffblocks (12), vorzugsweise mindestens 85 % der Oberfläche, insbesondere mindestens 95 % der Oberfläche durch die Folie (11) abgedeckt werden. Das erste Verbandmaterial umfasst erfindungsgemäß eine flexible, perforierte Folie, während das zweite Verbandmaterial einen porösen Polymerschaum umfasst. Die flexible Folie (11) umfasst auf ihrer zweiten Seite eine Vielzahl über die Fläche der Folie verteilter, dreidimensional ausgestalteter Perforationen. Die Folie (11) weist somit eine glatte erste Seite und eine aufgrund der in die Folie eingebrachten Perforationen (15) aufgeraute zweite Seite auf. Die Figuren 1c und 1d zeigen Zeichnungen nach mikroskopischen Aufnahmen einer für die hier dargestellte Ausführungsform geeigneten perforierten Polyethylen-Folie (11) in der Aufsicht auf die zweite Seite (Figur 1d) und von der Seite (Figur 1d). Figur 1e zeigt das Oberflächendetail aus Figur 1d in schematischer Darstellung. Aus den Figuren 1c, 1d und 1e ist erkennbar, dass kraterartig aus der Planebene der Folie herausragende Mikrostrukturen (16), welche der zweiten Oberfläche der Folie (11) eine raue Oberflächenbeschaffenheit verleihen, vorhanden sind. Bei den kraterartigen Strukturen (16), welche eine Höhe (senkrecht zur Ebene der Folie gemessene maximale Erstreckung der dreidimensionalen Struktur) von ungefähr 400 µm aufweisen, handelt es sich um Perforationsränder. Im Inneren der Mikrostrukturen (16) sind die Folie durchdringende Öffnungen beziehungsweise Kanäle (15) vorhanden. Der Durchmesser der Öffnungen beträgt jeweils ca. 0,3 mm. Die in Figur 1a beziehungsweise 1 c gezeigte Folie weist eine offene Fläche von 21 % auf, so dass eine wirksame Ableitung der in einer Wundkavität während der Therapie abgegebenen Wundexsudate gewährleistet werden kann. Die Figuren 1c und 1d zeigen somit ein Detail der zweiten Oberfläche der in Figur 1 a nur schematisch dargestellten flexiblen Folie (11). Figur 1b zeigt eine Zeichnung nach einer mikroskopischen Aufnahme der Oberfläche des offenzelligen Polymerschaums (12). In Figur 1b sind Schaumstoffstege (14) und Hohlräume (13) erkennbar. Die Schaumstoffstege (14) und die Hohlräume (13) bilden an der Schaumstoffoberfläche eine dreidimensional ausgestaltete Mikrostruktur, welche mit den auf der zweiten Seite der flexiblen Folie (11) vorhandenen kraterförmigen Mikrostrukturen (16) eine haftende Verbindung eingehen können, wenn die zweite Oberfläche der Folie (11) mit der ersten Oberfläche des Schaumstoffs (12) in Kontakt gebracht wird. Bei dem porösen Polymerschaum handelt es sich vorzugsweise um einen offenzelligen Schaumstoff aus Polyurethan oder aus Polyvinyl-Alkohol (PVA), insbesondere aus Polyester-Polyurethan. Die äußere Form des Schaumstoffblocks kann von dem Anwender an die zu behandelnde Wundkavität angepasst werden. Vorzugsweise liegt der Schaumstoffblock in Form eines schlauchförmigen Zylinders oder in Form eines Quaders vor.

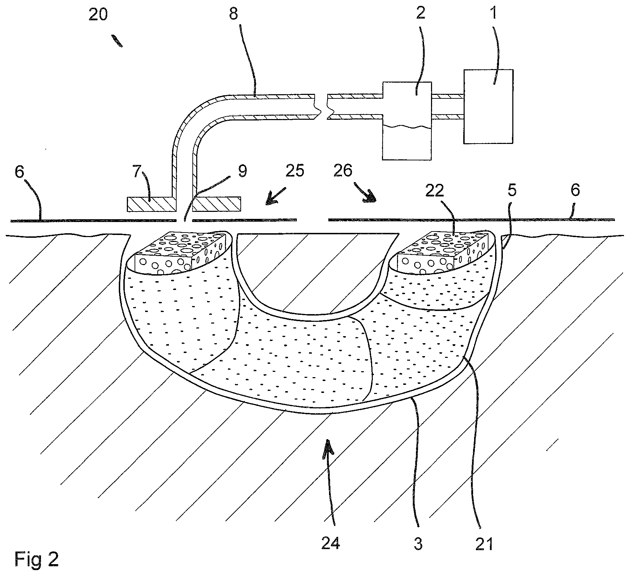

Figur 2 zeigt die Verwendung eines Verbundes aus erstem und zweitem Verbandmaterial bei der Behandlung einer tunnelartig ausgebildeten Wundkavität (24) mittels Unterdruck. Der durch eine Folie (21) zumindest teilweise umhüllte, schlauchförmig ausgebildete Schaumstoffblock (22) wurde vollständig in die Wundröhre (24) eingebracht. Die Folie (21) fungiert hierbei als Wundkontaktschicht, welche mit dem Wundgrund (3), also mit den Wandungen der Kavität, in unmittelbarer Berührung steht. Die Wundstellen (25, 26) an den Austrittsöffnungen der Kavität (24) werden mit einem luftundurchlässigen Abdeckfilm (6), beispielsweise mit einem transparenten Filmverband, unterdruckdicht versiegelt, wobei der Abdeckfilm (6) in der Wundumgebung auf der intakten Haut adhäsiv befestigt wird. Vorzugsweise handelt es sich bei dem zur Abdichtung des Unterdruckverbandes verwendeten Abdeckfilm (6) um einen selbstklebend beschichteten Polyurethanfilm, beispielsweise um das kommerziell erhältliche Produkt Hydrofilm^{®} (Paul Hartmann AG, Deutschland). In den Abdeckfilm (6) wird an der ersten versiegelten Wundstelle (25), d.h. an einer ersten Austrittstelle der tunnelförmigen Kavität, eine Öffnung (9) mit einem Durchmesser von ungefähr 0,5 cm eingebracht. Ein Unterdruckanschlussmittel (Port, 7), welches mit einer Unterdruckleitung (8) verbunden ist, wird über der Öffnung (9) befestigt, so dass eine Unterdruckkommunikation zwischen dem Lumen der Unterdruckleitung (8) und dem Wundraum hergestellt werden kann. Nach einem Aktivieren der Unterdruckquelle (1) kann Unterdruck im Wundraum hergestellt und Wundsekrete in den Behälter (2) gesaugt werden. Es wäre auch denkbar, an beiden Wundstellen (25, 26) jeweils ein Unterdruckanschlussstück anzubringen, so dass die durchgehende röhrenförmige Wundkavität (24) beidseitig mit Unterdruck beaufschlagt werden kann. Eine weitere, in Figur 2 nicht dargestellte Variante der Unterdruckapplikation besteht darin, an der ersten Wundstelle (25) ein Unterdruckanschlussstück anzubringen, während an der zweiten Wundstelle (26) eine kleine Öffnung in den Abdeckfilm eingebracht wird (in Figur 2 nicht dargestellt). Bei der Unterdruckapplikation kann so ein gerichteter Fluidstrom durch die Wundröhre (24) zum Unterdruckanschlussstück (7) hin erzeugt werden. Die Öffnung sollte hierbei so beschaffen sein, dass nur eine geringe Luftmenge in den Wundraum (Kavität 24) eintreten kann. Vorzugsweise erfolgt hierbei der Lufteintritt über einen Filter (in Figur 2 nicht dargestellt), welcher über der Öffnung angeordnet ist, so dass nur sterile Außenluft in den Wundraum gelangen kann. Gemäß einer weiteren vorteilhaften Ausführungsform ist über der Öffnung neben dem Filter zusätzlich ein Ventil angebracht, welches eine gezielte Regulierung des Lufteintrittes erlaubt. Während das Ventil geschlossen ist, kann der Wundraum nur über das Unterdruckanschlussstück kommunizieren. Der Unterdruck im Wundraum entspricht dann weitgehend dem durch die Pumpe bereitgestellten Unterdruck. Nach einer Ventilöffnung kann eine geringe Menge Außenluft über eine Öffnung der Tunnelröhre in den Wundkavität eintreten. Die Luft wird dann im Inneren des Verbundes aus erstem und zweitem Verbandmaterial durch die Wundröhre geleitet und entweicht über das Unterdruckanschlussstück. Eine derartige Durchspülung der Kavität mit Außenluft kann die Ableitung von Wundexsudat fördern, wobei zur Vermeidung einer übermäßigen Austrocknung des Wundgrundes (3) in der Kavität die Luftspülung vorzugsweise nur vorübergehend erfolgt, beispielsweise indem das Ventil mehrmals am Tag (beispielsweise dreimal pro Tag) für einige Minuten (beispielsweise 5 min) geöffnet und dann wieder geschlossen wird.

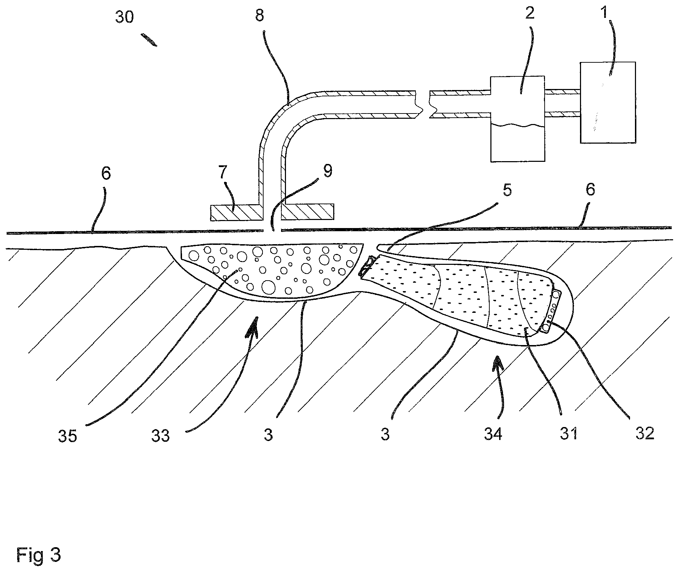

Figur 3 zeigt die Verwendung eines Verbundes aus erstem und zweitem Verbandmaterial bei der Behandlung einer blind endenden, taschenförmig ausgebildeten Wundkavität (34) mittels Unterdruck. Bei dem in Figur 3 gezeigten Beispiel ist ein nach außen hin offener Wundbereich (33) vorhanden, von dessen seitlicher Wandung der Wundkanal (34) abzweigt. Der durch eine Folie (31) zumindest teilweise umhüllte, schlauchförmig ausgebildete Schaumstoffblock (32) wird in die Kavität (34), also in den unterminierten, subdermalen Bereich des Wundraums eingebracht. Hierbei ist es vorteilhaft, wenn der Endabschnitt des Verbundes aus Schaumstofffolie geringfügig, beispielsweise 0,5 bis 2 cm, in den offenen Wundbereich (33) hineinragt, so dass die Wundtasche (34) während der Unterdruckapplikation durch den umhüllten Schaumstoff offen gehalten wird. Die perforierte Folie (31) fungiert hierbei als Wundkontaktschicht, welche mit dem Wundgrund (3), also mit den Wandungen der Kavität, in unmittelbarer Berührung steht. Die zu dem nach außen hin offenen Wundbereich (33) zeigende Oberfläche des Schaumstoffblocks (32) liegt vorzugsweise frei, d.h. nicht mit der Folie (31) überzogen, vor. In den nach außen hin offenen Wundbereich (33) wird ein weiterer, nicht durch eine Folie umhüllter poröser Polymerschaumstoff (35) eingebracht, welcher an Größe und Umfang des offenen Wundbereichs (33) angepasst ist. Die seitliche Oberfläche des durch Folie (31) umhüllten Schaumstoffblocks (32) sollte möglichst in einem direkten Kontakt mit dem weiteren Polymerschaumstoff (35) stehen, um eine effiziente Ableitung von aus der Kavität (34) abgesaugtem Wundexsudat zu gewährleisten. Zwischen dem weiteren Polymerschaumstoff (35) und dem Wundgrund kann bei Bedarf eine Wundkontaktschicht (in Figur 3 nicht dargestellt) eingelegt werden. Als Wundkontaktschicht kann beispielsweise gleichfalls eine perforierte Folie verwendet werden. Alternativ können aus dem Stand der Technik bekannte, zur Verwendung als Wundkontaktschicht geeignete Verbandmaterial zur Anwendung kommen, beispielsweise Salbenkompressen, insbesondere Salbenkompressen, welche eine antimikrobiell wirksame Substanz enthalten. Eine als Wundkontaktschicht im Zusammenhang mit der vorliegenden Erfindung geeignete Salbenkompresse ist das kommerziell erhältliche Produkt Atrauman Ag^{®} (Paul Hartmann AG, Deutschland). Die Wundstelle wird, wie in Figur 2 beschrieben, mit einem luftundurchlässigen Abdeckfilm (6), beispielsweise mit einem transparenten Filmverband, unterdruckdicht versiegelt, wobei der Abdeckfilm (6) in der Wundumgebung auf der intakten Haut adhäsiv befestigt wird. In den Abdeckfilm (6) wird eine Öffnung (9) mit einem Durchmesser von ungefähr 0,5 cm eingebracht. Ein Unterdruckanschlussmittel (Port, 7), welches mit einer Unterdruckleitung (8) verbunden ist, wird über der Öffnung (9) befestigt, so dass eine Unterdruckkommunikation zwischen dem Lumen der Unterdruckleitung (8) und dem Wundraum hergestellt werden kann. Nach einem Aktivieren der Unterdruckquelle (1) kann Unterdruck im Wundraum hergestellt und Wundsekrete in den Behälter (2) gesaugt werden. Gemäß einer weiteren, in Figur 3 nicht dargestellten Ausführungsform, könnte in das Innere des Schaumstoffblocks vor der Applikation der Wundauflage eine Drainageleitung eingebracht werden.

## Patentansprüche

1. Verbandset, geeignet zur Verwendung bei der Behandlung von Wundkavitäten, insbesondere zur Verwendung bei der Behandlung von Wundkavitäten mittels Unterdruck, umfassend
i) ein erstes Verbandmaterial als Wundkontaktschicht, umfassend eine flexible, perforierte Folie (11, 21, 31) mit einer ersten und einer zweiten Seite, wobei die in der Folie vorhandenen Perforationen (15) derart beschaffen sind, dass die Perforationsränder (16) von der zweiten Seite der Folie (11, 21, 31) abstehen, so dass auf der zweiten Seite der Folie (11, 21, 31) dreidimensional ausgestaltete Strukturen vorhanden sind und wobei die erste Seite zum Inkontaktbringen mit einem Wundgrund (3), insbesondere mit der inneren Oberfläche einer Wundröhre, vorgesehen ist,
ii) ein separat bereit gestelltes zweites Verbandmaterial zum Einbringen in eine Wundkavität, umfassend einen porösen Polymerschaum (12, 22, 32), wobei es sich bei dem Polymerschaum (12, 22, 32) um einen offenzelligen Polymerschaum handelt, welcher an oder nahe seiner Oberfläche Stege (14) umfasst und/oder welcher auf seiner Oberfläche zur Oberfläche hin offene Hohlräume (13) umfasst, und wobei die Stege (14) und/oder Hohlräume (13) dreidimensional ausgestaltete Strukturen bilden,
**dadurch gekennzeichnet,**
**dass** das erste Verbandmaterial über eine Fläche verfügt, welche ausreichend ist, mindestens 75 % der Oberfläche des zweiten Verbandmaterials zu umhüllen,
und **dass** die auf der zweiten Seite des ersten Verbandmaterials vorhandenen Strukturen mit den auf der Oberfläche des zweiten Verbandmaterials vorhandenen Strukturen eine haftende Verbindung eingehen können,
wobei die zweite Seite der Folie (11, 21, 31) zum teilweisen oder vollständigen Umhüllen der Oberfläche des Polymerschaums (12, 22, 32) unmittelbar vor der Wundbehandlung vorgesehen ist, so dass nach dem Einbringen des Verbundes aus erstem und zweitem Verbandmaterial in eine Wundkavität aufgrund der haftenden Verbindung zwischen dem ersten Verbandmaterial und dem zweiten Verbandmaterial ein Verschieben des ersten Verbandmaterials gegenüber dem zweiten Verbandmaterial während der Therapie weitestgehend vermieden werden kann und/oder das gleichzeitige Entfernen von dem ersten Verbandmaterial und dem zweitem Verbandmaterial erleichtert wird, wobei zum Verschieben des ersten Verbandmaterials im nassen Zustand gegen das zweite Verbandmaterial im nassen Zustand eine statische Gleitreibungskraft Fₛ, gemessen nach DIN EN ISO 8235, von mindestens 3 N erforderlich ist und/oder wobei zum Verschieben des ersten Verbandmaterials im trockenen Zustand gegen das zweite Verbandmaterial im trockenen Zustand eine statische Gleitreibungskraft Fₛ von mindestens 6 N, gemessen nach DIN EN ISO 8235, erforderlich ist.

2. Verbandset nach Anspruch 1, wobei die Fläche der ersten Seite des ersten Verbandmaterials mindestens 80 % und höchstens 5000 %, insbesondere mindestens 120 % und höchstens 2000 % der Oberfläche des zweiten Verbandmaterials beträgt.

3. Verbandset nach einem der vorangehenden Ansprüche, wobei das zweite Verbandmaterial in Form eines schlauchförmig ausgebildeten Zylinders vorliegt, dessen Höhe (h) mindestens das dreifache, insbesondere mindestens das fünffache des Durchmessers beträgt.

4. Verbandset nach einem der Ansprüche 1 bis 2, wobei das zweite Verbandmaterial in Form eines in die Länge gestreckten Quaders mit den Seiten (a; b; c) vorliegt, dessen Verhältnis der Seitenlängen der durch die Seiten (a; b) gebildeten Querschnittsfläche 0,5 < (a : b) < 2 beträgt, und wobei die längere Seite c größer als die Diagonale der Querschnittsfläche ist.

5. Verbandset nach einem der vorangehenden Ansprüche, wobei es sich bei den auf der zweiten Seite der Folie (11, 21,31) vorhandenen dreidimensional ausgestalteten Strukturen um kraterförmig geformte Strukturen handelt.

6. Verbandset nach einem der vorangehenden Ansprüche, wobei die in der Folie (11, 21, 31) vorhandenen Perforationen (15) einen offenen Durchmesser von mindestens 0,2 mm und höchstens 0,4 mm aufweisen.

7. Verbandset nach einem der vorangehenden Ansprüche, wobei das zweite Verbandmaterial einen in das Innere des porösen Polymerschaums (12, 22, 32) eingeführten Drainageschlauch umfasst, so dass der Schaum (12, 22, 32) mit Unterdruck beaufschlagt und Fluide aus dem Schaum (12, 22, 32) heraus zu einer Unterdruckquelle (1) hin befördert werden können.

8. Verbandset nach einem der vorangehenden Ansprüche, wobei an dem ersten Verbandmaterial und/oder an dem zweiten Verbandmaterial mindestens eine zusätzliche Komponente vorhanden ist, welche die haftende Verbindung zwischen dem ersten Verbandmaterial und dem zweiten Verbandmaterial weiter verstärken kann, wobei es sich bei der zusätzlichen Komponente um eine adhäsive Beschichtung handelt.

9. Verbandset nach einem der vorangehenden Ansprüche, wobei das erste und das zweite Verbandmaterial jeweils einzeln steril konfektioniert vorliegen.

10. Verbandset nach Anspruch 9, weiter umfassend eine luftdichte Abdeckfolie (6) zum luftdichten Versiegeln des Wundgebietes und optional ein Unterdruckanschlussstück (7) zum Beaufschlagen des Wundraumes mit Unterdruck.

11. Verbund aus mindestens zwei Komponenten, geeignet zur Verwendung bei der Behandlung von Wundkavitäten, insbesondere zur Verwendung bei der Behandlung von Wundkavitäten mittels Unterdruck, umfassend
i) ein erstes Verbandmaterial als Wundkontaktschicht, umfassend eine flexible, perforierte Folie (11, 21, 31) mit einer ersten und einer zweiten Seite, wobei die in der Folie (11, 21, 31) vorhandenen Perforationen (15) derart beschaffen sind, dass die Perforationsränder (16) von der zweiten Seite der Folie (11, 21, 31) abstehen, so dass auf der zweiten Seite der Folie (11, 21, 31) dreidimensional ausgestaltete Strukturen vorhanden sind und wobei die erste Seite zum Inkontaktbringen mit einem Wundgrund (3), insbesondere mit der inneren Oberfläche einer Wundröhre, vorgesehen ist,
ii) ein zweites Verbandmaterial zum Einbringen in eine Wundkavität, umfassend einen porösen Polymerschaum (12, 22, 32), wobei es sich bei dem Polymerschaum (12, 22, 32) um einen offenzelligen Polymerschaum handelt, welcher an oder nahe seiner Oberfläche Stege (14) umfasst und/oder welcher auf seiner Oberfläche zur Oberfläche hin offene Hohlräume (13) umfasst, und wobei die Stege (14) und/oder Hohlräume (13) dreidimensional ausgestaltete Strukturen bilden,
**dadurch gekennzeichnet,**
**dass** die auf der zweiten Seite des ersten Verbandmaterials vorhandenen Strukturen mit den auf der Oberfläche des zweiten Verbandmaterials vorhandenen Strukturen eine haftende Verbindung eingehen können,
und **dass** die Folie (11, 21, 31) auf mindestens 75 % der Oberfläche des Polymerschaums (12, 22, 32) vorliegt und somit eine Umhüllung des Polymerschaums (12, 22, 32) bildet, wobei die Folie (11, 21,31) mit ihrer zweiten Seite in Kontakt mit dem Polymerschaum (12, 22, 32) steht,
so dass nach dem Einbringen des Verbundes aus erstem und zweitem Verbandmaterial in eine Wundkavität aufgrund der haftenden Verbindung zwischen dem ersten Verbandmaterial und dem zweiten Verbandmaterial ein Verschieben des ersten Verbandmaterials gegenüber dem zweiten Verbandmaterial während der Therapie weitestgehend vermieden werden kann und/oder das gleichzeitige Entfernen von dem ersten Verbandmaterial und dem zweitem Verbandmaterial erleichtert wird, wobei zum Verschieben des ersten Verbandmaterials im nassen Zustand gegen das zweite Verbandmaterial im nassen Zustand eine statische Gleitreibungskraft Fₛ, gemessen nach DIN EN ISO 8235, von mindestens 3 N erforderlich ist und/oder wobei zum Verschieben des ersten Verbandmaterials im trockenen Zustand gegen das zweite Verbandmaterial im trockenen Zustand eine statische Gleitreibungskraft Fₛ von mindestens 6 N, gemessen nach DIN EN ISO 8235, erforderlich ist.

12. Verbandset nach einem oder mehreren der Ansprüche 1 bis 10 oder Verbund nach Anspruch 11, wobei die in der Folie vorhandenen Perforationen (15) über die Fläche verteilt vorliegen.

13. Vorrichtung, geeignet zur Verwendung bei der Behandlung von Wundkavitäten mittels Unterdruck, umfassend ein Verbandset nach einem der Ansprüche 1 bis 10, eine luftdichte Abdeckfolie (6), ein Unterdruckanschlussstück (7), eine Unterdruckleitung (8), eine Unterdruckquelle (1) sowie optional einen Behälter (2) für die abgesaugten Wundfluide.

## Claims

1. Bandage set suitable for use in the treatment of wound cavities, especially for use in the treatment of wound cavities by means of negative pressure, comprising
i) a first bandage material as wound contact layer, comprising a flexible, perforated film (11, 21, 31) having a first and a second side, the perforations (15) present in the film being provided in such a way that the perforation edges (16) protrude from the second side of the film (11, 21, 31), with three-dimensional structures being present on the second side of the film (11, 21, 31), and the first side being intended for contacting with a wound base (3), more particularly the internal surface of a wound tube,
ii) a separately provided second bandage material for introduction into a wound cavity, comprising a porous polymer foam (12, 22, 32), the polymer foam (12, 22, 32) being an open-cell polymer foam which comprises struts (14) on or close to its surface and/or which comprises, on its surface, hollow spaces (13) open toward the surface, and the struts (14) and/or hollow spaces (13) forming three-dimensional structures,
**characterized in that**
the first bandage material has a surface area sufficient to envelop at least 75% of the surface of the second bandage material,
and the structures present on the second side of the first bandage material can form an adhesive connection with the structures present on the surface of the second bandage material,
the second side of the film (11, 21, 31) being intended for partial or complete envelopment of the surface of the polymer foam (12, 22, 32) immediately before the wound treatment, and so, after introduction of the composite composed of first and second bandage material into a wound cavity, movement of the first bandage material with respect to the second bandage material can be avoided as far as possible during the therapy and/or simultaneous removal of the first bandage material and the second bandage material is facilitated owing to the adhesive connection between the first bandage material and the second bandage material, wherein a static sliding friction force Fₛ, measured in accordance with DIN EN ISO 8235, of at least 3 N is required in order to move the first bandage material in the wet state against the second bandage material in the wet state and/or wherein a static sliding friction force Fₛ of at least 6 N, measured in accordance with DIN EN ISO 8235, is required in order to move the first bandage material in the dry state against the second bandage material in the dry state.

2. Bandage set according to Claim 1, wherein the surface area of the first side of the first bandage material is at least 80% and not more than 5000%, in particular at least 120% and not more than 2000%, of the surface of the second bandage material.

3. Bandage set according to either of the preceding claims, wherein the second bandage material is present in the form of a hose-shaped cylinder, the height (h) of which is at least three times, in particular at least five times, the diameter.

4. Bandage set according to either of Claims 1 and 2, wherein the second bandage material is present in the form of a cuboid stretched in length and having sides (a; b; c), the cuboid having a ratio of the side lengths of the cross-sectional area formed by sides (a; b) of 0.5 < (a : b) < 2, and wherein the longer side c is greater than the diagonal of the cross-sectional area.

5. Bandage set according to any of the preceding claims, wherein the three-dimensional structures present on the second side of the film (11, 21, 31) are crater-shaped structures.

6. Bandage set according to any of the preceding claims, wherein the perforations (15) present in the film (11, 21, 31) have an open diameter of at least 0.2 mm and not more than 0.4 mm.

7. Bandage set according to any of the preceding claims, wherein the second bandage material comprises a drainage hose introduced into the interior of the porous polymer foam (12, 22, 32), making it possible to apply negative pressure to the foam (12, 22, 32) and to transport fluids out of the foam (12, 22, 32) toward a negative-pressure source (1).

8. Bandage set according to any of the preceding claims, wherein at least one additional component which can further strengthen the adhesive connection between the first bandage material and the second bandage material is present on the first bandage material and/or on the second bandage material, wherein the additional component is an adhesive coating.

9. Bandage set according to any of the preceding claims, wherein the first and the second bandage material are both individually available off-the-shelf and in sterile form.

10. Bandage set according to Claim 9, further comprising an airtight covering film (6) for airtight sealing of the wound area and optionally a negative-pressure connecting piece (7) for applying negative pressure to the wound space.

11. Composite composed of at least two components, suitable for use in the treatment of wound cavities, especially for use in the treatment of wound cavities by means of negative pressure, comprising
i) a first bandage material as wound contact layer, comprising a flexible, perforated film (11, 21, 31) having a first and a second side, the perforations (15) present in the film (11, 21, 31) being provided in such a way that the perforation edges (16) protrude from the second side of the film (11, 21, 31), with three-dimensional structures being present on the second side of the film (11, 21, 31), and the first side being intended for contacting with a wound base (3), more particularly the internal surface of a wound tube,
ii) a second bandage material for introduction into a wound cavity, comprising a porous polymer foam (12, 22, 32), the polymer foam (12, 22, 32) being an open-cell polymer foam which comprises struts (14) on or close to its surface and/or which comprises, on its surface, hollow spaces (13) open toward the surface, and the struts (14) and/or hollow spaces (13) forming three-dimensional structures,
**characterized in that**
the structures present on the second side of the first bandage material can form an adhesive connection with the structures present on the surface of the second bandage material,
and the film (11, 21, 31) is present on at least 75% of the surface of the polymer foam (12, 22, 32) and thus forms an envelopment of the polymer foam (12, 22, 32), the film (11, 21, 31), with its second side, being in contact with the polymer foam (12, 22, 32),
and so, after introduction of the composite composed of first and second bandage material into a wound cavity, movement of the first bandage material with respect to the second bandage material can be avoided as far as possible during the therapy and/or simultaneous removal of the first bandage material and the second bandage material is facilitated owing to the adhesive connection between the first bandage material and the second bandage material, wherein a static sliding friction force Fₛ, measured in accordance with DIN EN ISO 8235, of at least 3 N is required in order to move the first bandage material in the wet state against the second bandage material in the wet state and/or wherein a static sliding friction force Fₛ of at least 6 N, measured in accordance with DIN EN ISO 8235, is required in order to move the first bandage material in the dry state against the second bandage material in the dry state.

12. Bandage set according to one or ore of Claims 1 to 10 or composite according to Claim 11, wherein the perforations (15) present in the film are present distributed across the surface.

13. Apparatus suitable for use in the treatment of wound cavities by means of negative pressure, comprising a bandage set according to any of Claims 1 to 10, an airtight covering film (6), a negative-pressure connecting piece (7), a negative-pressure line (8), a negative-pressure source (1) and also optionally a container (2) for the aspirated wound fluids.

## Revendications

1. Nécessaire de pansement, approprié à l'utilisation dans le traitement de cavités de plaies, en particulier à l'utilisation dans le traitement de cavités de plaies au moyen d'une dépression, comprenant
i) un premier matériau de pansement en tant que couche en contact avec la plaie, comprenant un film perforé flexible (11, 21, 31) comportant une première face et une seconde, les perforations (15) présentes dans le film étant conçues de manière que les bords (16) des perforations s'écartent de la seconde face du film (11, 21, 31), de sorte que sur la seconde face du film (11, 21, 31) sont présentes des structures en configuration tridimensionnelle et la première face étant prévue pour la mise en contact avec un fond de plaie (3), en particulier avec la surface interne d'un tube de plaie,
ii) un deuxième matériau de pansement déjà préparé séparément, destiné à l'introduction dans une cavité de plaie, comprenant une mousse polymère poreuse (12, 22, 32), la mousse polymère (12, 22, 32) consistant en une mousse polymère à cellules ouvertes, qui comprend à ou au voisinage de sa surface des travées (14) et/ou qui comprend à sa surface des espaces vides (13) ouverts en direction de la surface, et les travées (14) et/ou les espaces vides (13) formant des structures en configuration tridimensionnelle,
**caractérisé en ce que**
le premier matériau de pansement est doté d'une surface qui est suffisante pour entourer au moins 75 % de la surface du deuxième matériau de pansement,
et **en ce que** les structures présentes sur la deuxième face du premier matériau de pansement peuvent entrer en un assemblage adhérant avec les structures présentes à la surface du deuxième matériau de pansement,
la seconde face du film (11, 21, 31) étant prévue pour entourer partiellement ou complètement la surface de la mousse polymère (12, 22, 32) immédiatement avant le traitement de la plaie, de sorte qu'après l'introduction du composite de premier et deuxième matériau de pansement dans une cavité de plaie, en raison de l'assemblage adhérant entre le premier matériau de pansement et le deuxième matériau de pansement un déplacement du premier matériau de pansement vis-à-vis du deuxième matériau de pansement pendant le traitement peut être dans une large mesure évité et/ou l'enlèvement simultané du premier matériau de pansement et du deuxième matériau de pansement est facilité, une force statique de frottement de glissement Fₛ, mesurée selon DIN EN ISO 8235, d'au moins 3 N étant requise pour le déplacement du premier matériau de pansement à l'état humide contre le deuxième matériau de pansement à l'état humide et/ou une force statique de frottement de glissement Fₛ, mesurée selon DIN EN ISO 8235, d'au moins 6 N étant requise pour le déplacement du premier matériau de pansement à l'état sec contre le deuxième matériau de pansement à l'état sec.

2. Nécessaire de pansement selon la revendication 1, dans lequel la surface de la première face du premier matériau de pansement représente au moins 80 % et au maximum 5000 %, en particulier au moins 120 % et au maximum 2000 % de la surface du deuxième matériau de pansement.

3. Nécessaire de pansement selon l'une quelconque des revendications précédentes, dans lequel le deuxième matériau de pansement se trouve sous forme d'un cylindre configuré en tuyau souple, dont la hauteur (h) représente eu moins le triple, en particulier au moins le quintuple du diamètre.

4. Nécessaire de pansement selon l'une quelconque des revendications 1 et 2, dans lequel le deuxième matériau de pansement se trouve sous forme d'un parallélépipède étiré en longueur, comportant les côtés (a ; b ; c), dont le rapport des longueurs des côtés de la surface de section transversale formée par les côtés (a ; b) vaut 0,5 < (a : b) < 2, et dans lequel le plus long côté c est plus grand que la diagonale de la surface de section transversale.

5. Nécessaire de pansement selon l'une quelconque des revendications précédentes, dans lequel pour ce qui est des structures en configuration tridimensionnelle présentes sur la seconde face du film (11, 21, 31) il s'agit de structures moulées en forme de cratère.

6. Nécessaire de pansement selon l'une quelconque des revendications précédentes, dans lequel les perforations (15) présentes dans le film (11, 21, 31) présentent un diamètre ouvert d'au moins 0,2 mm et d'au maximum 0,4 mm.

7. Nécessaire de pansement selon l'une quelconque des revendications précédentes, dans lequel le deuxième matériau de pansement comprend un tuyau souple de drainage inséré à l'intérieur de la mousse polymère poreuse (12, 22, 32), de sorte que la mousse (12, 22, 32) est soumise à une dépression et des fluides peuvent, à partir de la mousse (12, 22, 32), être transportés vers une source de dépression (1).

8. Nécessaire de pansement selon l'une quelconque des revendications précédentes, dans lequel sur le premier matériau de pansement et/ou sur le deuxième matériau de pansement est présent au moins un composant supplémentaire qui peut renforcer davantage l'assemblage adhérant entre le premier matériau de pansement et de deuxième matériau de pansement, le composant supplémentaire consistant en un revêtement adhésif.

9. Nécessaire de pansement selon l'une quelconque des revendications précédentes, dans lequel le premier matériau de pansement et le deuxième matériau de pansement se trouvent chacun individuellement conditionnés stériles.

10. Nécessaire de pansement selon la revendication 9, comprenant en outre un film de recouvrement (6) étanche à l'air, destiné à sceller de façon étanche à l'air la zone de la plaie et en option une pièce de raccord de dépression (7) destiné à l'application d'une dépression dans l'espace de la plaie.

11. Composite à base d'au moins deux composants, approprié à l'utilisation dans le traitement de cavités de plaies, en particulier à l'utilisation dans le traitement de cavités de plaies au moyen d'une dépression, comprenant
i) un premier matériau de pansement en tant que couche en contact avec la plaie, comprenant un film perforé flexible (11, 21, 31) comportant une première face et une seconde, les perforations (15) présentes dans le film (11, 21, 31) étant conçues de manière que les bords (16) des perforations s'écartent de la seconde face du film (11, 21, 31), de sorte que sur la seconde face du film (11, 21, 31) sont présentes des structures en configuration tridimensionnelle et la première face étant prévue pour la mise en contact avec un fond de plaie (3), en particulier avec la surface interne d'un tube de plaie,
ii) un deuxième matériau de pansement destiné à l'introduction dans une cavité de plaie, comprenant une mousse polymère poreuse (12, 22, 32), la mousse polymère (12, 22, 32) consistant en une mousse polymère à cellules ouvertes, qui comprend à ou au voisinage de sa surface des travées (14) et/ou qui comprend à sa surface des espaces vides (13) ouverts en direction de la surface, et les travées (14) et/ou les espaces vides (13) formant des structures en configuration tridimensionnelle,
**caractérisé en ce que**
les structures présentes sur la seconde face du premier matériau de pansement peuvent entrer en un assemblage adhérant avec les structures présentes à la surface du deuxième matériau de pansement,
et **en ce que** le film (11, 21, 31) est présent sur au moins 75 % de la surface de la mousse polymère (12, 22, 32) et forme par conséquent un enveloppement de la mousse polymère (12, 22, 32), le film (11, 21, 31) étant en contact par sa seconde face avec la mousse polymère (12, 22, 32),
de sorte qu'après l'introduction du composite à base du premier et deuxième matériau de pansement dans une cavité de plaie, en raison de l'assemblage adhérant entre le premier matériau de pansement et le deuxième matériau de pansement un déplacement du premier matériau de pansement vis-à-vis du deuxième matériau de pansement pendant le traitement peut être dans une large mesure évité et/ou l'enlèvement simultané du premier matériau de pansement et du deuxième matériau de pansement est facilité, une force statique de frottement de glissement Fₛ, mesurée selon DIN EN ISO 8235, d'au moins 3 N étant requise pour le déplacement du premier matériau de pansement à l'état humide contre le deuxième matériau de pansement à l'état humide et/ou une force statique de frottement de glissement Fₛ d'au moins 6 N, mesurée selon DIN EN ISO 8235, étant requise pour le déplacement du premier matériau de pansement à l'état sec contre le deuxième matériau de pansement à l'état sec.

12. Nécessaire de pansement selon une ou plusieurs des revendications 1 à 10 ou composite selon la revendication 11, dans lequel les perforations (15) présentes dans le film se trouvent réparties sur la surface.

13. Dispositif, approprié à l'utilisation dans le traitement de cavités de plaies au moyen de dépression, comprenant un nécessaire de pansement selon l'une quelconque des revendications 1 à 10, un film de recouvrement (6) étanche à l'air, une pièce de raccordement de dépression (7), un conduit de dépression (8), une source de dépression (1) ainsi qu'en option un récipient (2) pour les fluides de plaie aspirés.
